# EUROPEAN PATENT APPLICATION

(11) **EP 2 896 294 A1**
(43) Date of publication of application: **22.07.2015**
(21) Application number: 12884237.4
(22) Date of filing: 07.09.2012
(51) Int. Cl.: A23C 9/152, C12N 15/113, C12N 15/117

(54) **MICRORNA IN HUMAN MILK AND USE THEREOF**

(71) Applicant: Micromedmark Biotech Co., Ltd., Beijing 100006 (CN)
(72) Inventor: ZHANG, Chenyu, Beijing 100738 (CN); SI, Qin, Beijing 100738 (CN); XILIN, Qiqige, Beijing 100738 (CN); CHEN, Xi, Beijing 100738 (CN); ZHANG, Qipeng, Beijing 100738 (CN); ZENG, Ke, Beijing 100738 (CN); WU, Lan, Beijing 100738 (CN)
(74) Representative: Tischner, Oliver
(86) International application number: PCT/CN2012/081146
(87) International publication number: WO 2014/036726

(57) **Abstract**

Provided are a series of microRNAs stabilized in human mature milk and/or colostrum and a use thereof. The microRNAs comprise one or more microRNAs selected from those shown in tables A, B, C and D, and can be used to prepare infantal foods or food additives or supplements.

## Description

### Technical field

The present invention belongs to the field of biological product, and in particular the present invention relates to human milk microRNAs and application thereof.

### Background art

Breast milk has been recognized as the most desirable infant food. World Health Organization also points out that breast milk is the desirable food for the healthy growth and development of infants. Because breast milk contains various kinds of nutrient substances suitable for the growth and development of infants, and its nutritional ingredients are comprehensive and can more easily be absorbed. In addition, breast milk also contains nutritional ingredients conducive to immunity enhancement, such as lactoferrin (it can inhibit harmful bacteria which need iron), and active white blood cells, immune antibodies, and other immune factors for resisting infections. Colostrum contains, among others, large amount of immunoglobulins, which can protect infants from bacterial infections, so the infants are not susceptible to diseases such as pneumonia.

Breast milk substitute (such as infant formula milk) is to be selected when mother is incapable of lactation due to such reasons as the mother cannot feed with breast or poor milk secretion. The preparation of infant formula milk uses breast milk as a standard, approximating to it to the full extent through modification of cow milk, thus meeting the requirements for the digestion, absorption, and nutrition of infants.

International Breastfeeding Association points out that the existing formula milk is not only lack of such important ingredients as hydroxy acids, multiple mineral substances, hormones, non-protein nitrogen fractions including amino acids and nucleotides compared with breast milk, but also contains insufficient varieties of main proteins, fats, and vitamins, and not easy for the digestion and absorption by infants. In conclusion, the existing formula milk is approximate to breast milk as far as possible, but it is still far different from breast milk.

Therefore, in the field there is still a need for developing a milk product with its nutritional substances being more approximate to that of breast milk.

### Contents of the invention

One of the objects of the present invention is to provide a novel microRNA set, said microRNA can be used to prepare infant foods or food additives or supplements.

Another object of the present invention is to provide a series of microRNA expression profiles and application thereof, including the expression profiles of human milk microRNAs and uses thereof, the microRNA expression profiles specific to colostrum and uses thereof, and the microRNA expression profiles specific to mature breast milk and uses thereof.

In a first aspect of the present invention, provided is an artificial milk product, said milk product contains one or more kinds of human milk-derived microRNAs which are exogenously added, said human milk includes human colostrum or human mature breast milk.

In another preferred example, said microRNAs are selected from the group consisting of:
(a) microRNAs specific to human colostrum and listed in Table A;
(b) microRNAs specific to human mature breast milk and listed in Table B;
(c) common microRNAs existing in both human colostrum and mature breast milk and listed in Table C; and
and (d) microRNAs not existing in cow milk but existing in human colostrum or human mature breast milk, and listed in Table D;

Said Table A, Table B, Table C and Table D are as described in the section "microRNA expression profile".

In another preferred example, said milk product comprises colostrum product and mature breast milk product.

Said milk product includes milk powder, liquid milk, cheese, yogurt and the like.

In another preferred example, said milk product also contains one or more kinds of microRNAs which are added exogenously and selected from the group comprising:(e) the microRNAs listed in Table E; said Table E is as described in section "microRNA expression profile".

In another preferred example, said milk product comprises one or more characteristics selected from the following group:
(a1) two or more kinds of said exogenously added microRNAs are from Table A;
(a2) the varieties and contents of said exogenously added microRNAs are approximate or identical to the microRNA expression profiles of human colostrum;
(b1) two or more kinds of said exogenously added microRNAs are from Table B;
(b2) the varieties and contents of said exogenously added microRNAs are approximate or identical to the microRNA expression profiles of human mature breast milk;
(c1) two or more kinds of said exogenously added microRNAs are from Table C; and
(d1) two or more kinds of said exogenously added microRNAs are from Table D;

In another preferred example, said two or more kinds are 2-500 kinds, preferably 3-300 kinds, and more preferably 3-200 kinds.

In another preferred example, said artificial milk product is a milk product applicable to infants of 0-24 months old.

In another preferred example, said artificial milk product is a milk product applicable to population above 2 years old.

In a second aspect of the present invention, provided is an artificial food supplement, said food supplement contains bromatologically acceptable carriers and one or more human milk-derived microRNAs selected from the group consisting of:
(a) microRNAs specific to human colostrum and listed in Table A;
(b) microRNAs specific to human mature breast milk and listed in Table B;
(c) common microRNAs existing both in human colostrum and mature breast milk and listed in Table C; and
(d) microRNAs not existing in cow milk but existing in human colostrum or human mature breast milk, and listed in Table D;

Said Table A, Table B, Table C and Table D are as described in the first aspect of the present invention.

In another preferred example, said food supplement also contains one or more kinds of microRNAs selected from the group comprising: (e) the microRNAs listed in Table E.

In another preferred example, said food supplement has one or more of the following characteristics:
(a1) two or more kinds of said exogenously added microRNAs are from Table A;
(a2) the varieties and contents of said exogenously added microRNAs are approximate or identical to the microRNA expression profiles of human colostrum;
(b1) two or more kinds of said exogenously added microRNAs are from Table B;
(b2) the varieties and contents of said exogenously added microRNAs are approximate or identical to the microRNA expression profiles of human mature breast milk;
(c1) two or more kinds of said exogenously added microRNAs are from Table C; and
(d1) two or more kinds of said exogenously added microRNAs are from Table D;

In a third aspect of the present invention, provided is a method for identifying a milk product, said method comprises:
(1) providing a milk product sample;
(2) detecting varieties and contents of the microRNAs in said milk product sample; and
(3) comparing the varieties of microRNAs and contents thereof detected in step (2) with expression profiles (i.e., the varieties of microRNAs and the contents thereof) of the microRNAs in a colostrum sample and a mature breast milk sample which are used as controls;

Wherein, said milk product is identified as a colostrum product if the detected varieties of the microRNAs and the contents thereof are approximate or identical to the expression profiles of the microRNAs of the colostrum sample; and said milk product is identified as a mature breast milk product if the detected varieties of the microRNAs and the contents thereof are approximate or identical to the expression profiles of the microRNAs of the mature breast milk sample.

In another preferred example, said milk product (such as milk powder) comprises cow milk or a milk product derived therefrom, goat's milk or a milk product derived therefrom, and human milk.

In another preferred example, the expression profiles of the microRNAs in mature breast milk or colostrum are obtained through detecting the varieties of microRNAs in mature breast milk or colostrum and the contents thereof.

In another preferred example, the expression profiles of the microRNAs in said colostrum comprise two or more kinds of microRNAs listed in Table A and Table C; and the expression profiles of the microRNAs in said mature breast milk comprise the microRNAs listed in Table B and Table C.

In a fourth aspect of the present invention, provided is a method of manufacturing a milk product, said method comprises:
(1) providing a milk product material;
(2) detecting varieties of microRNAs in said milk product material and contents thereof thereby obtaining a first microRNA expression profile;
(3) comparing the varieties of microRNAs and the contents thereof detected in step (2) with expression profiles of the microRNAs in a colostrum sample or a mature breast milk sample which are used as controls; and
(4) based on comparison results, adding one or more kinds of microRNAs to said milk product material and/or removing one or more kinds of microRNAs from said milk product material to produce a milk product, wherein the expression profiles of the microRNAs in said milk product are more approximate or identical to the expression profiles of the microRNAs in the colostrum sample or the mature breast milk sample which are used as controls.

In another preferred example, said milk product material is cow milk, goat milk or a combination thereof.

In a fifth aspect of the present invention, provided is a method of manufacturing a milk product, said method comprises:
(1) providing a milk product material;
(2) adding one or more kinds of microRNAs to said milk product material and/or removing one or more kinds of microRNAs from said milk product material to produce the milk product, wherein the expression profiles of microRNAs in said milk product are more approximate or identical to the expression profiles of the microRNAs in a colostrum or a mature breast milk.

In another preferred example, said colostrum or mature breast milk is human colostrum or human mature breast milk.

In a sixth aspect of the present invention, provided is a method of manufacturing a food supplement, said method comprises:
(1) detecting varieties and contents of the microRNAs in the breast milk of a certain subject to obtain a first microRNA expression profile of the subject;
(2) comparing the detected varieties of microRNAs of the first expression profile and the contents thereof with the expression profiles of microRNAs in a normal colostrum sample or a normal mature breast milk sample used as control, so as to determine microRNAs which are lack or insufficient in the first expression profile and needed to be supplemented;
(3) preparing the food supplement, wherein said food supplement contains the microRNAs as determined in the foregoing step, which are lack or insufficient in the first expression profile and needed to be supplemented.

In another preferred example, in the preparation step, included is mixing a carrier acceptable in bromatology with one or more artificially synthesized microRNAs so as to form a food supplement.

In a seventh aspect of the present invention, provided is an isolated microRNA set, said microRNA set is composed of two or more kinds of human milk-derived microRNAs selected from the group consisting of:
(a) microRNAs specific to human colostrum and listed in Table A;
(b) microRNAs specific to human mature breast milk and listed in Table B;
(c) common microRNAs existing in both human colostrum and mature breast milk and listed in Table C; and
(d) microRNAs not existing in cow milk but existing in human colostrum or human mature breast milk, and listed in Table D;

Said Table A, Table B, Table C and Table D are as described in the first aspect of the present invention.

In another preferred example, said microRNA set is composed of 2-500 kinds (preferably 5-300 kinds, and more preferably 5-100 kinds) of microRNAs.

In an eighth aspect of the present invention, provided is an artificial milk product, food or food supplement, which comprises the isolated microRNA set described in the seventh aspect of the present invention as an effective ingredient.

In another preferred example, the microRNA expression profiles of said artificial milk product or food supplement are approximate or identical to the expression profiles of the microRNAs in a normal human colostrum sample or human mature breast milk sample.

It should be understood that all of the various technical features described above and the various technical features specifically described hereinafter (such as examples) can be combined with one another within the scope of the present invention, so as to form new or preferred technical solutions. Due to space limitations, this is no longer tired out one by one.

### Description of drawings

Figure 1 is the differential expression analysis of the microRNAs in human colostrum and mature breast milk.
Figure 2 is the luciferase activity of the HEK-293 cells transfected with Pre-miR-19a and 3'-ESR1-wt.
Figure 3 is the Western blot result for the expression of ESR1 in GFP positive SK-N-BE (2) cells transfected with lentivirus expression vector (Lenti-ESR1) and blank control.
Figure 4 is the flow cytometry analysis result for the cell proliferation after transfecting the cells with Lenti-ESR1 and blank control.

### Particular embodiments

Through long-term deep research, the inventors have unexpectedly found that there are differences in the varieties and contents of the microRNAs in human milk and cow milk, or the milk secreted by a person in different periods (that is colostrum and mature breast milk). Multiple expression profiles of microRNAs of human milk are formed through screening and comparing the varieties or contents of the microRNAs of cow milk, human colostrum and human mature breast milk by the inventors, which have excellent significance in directing the preparation or staging of infant food formulations. The present invention is accomplished by the inventors on this basis.

### microRNAs

Micro ribonucleic acids (microRNA, miRNA or miR for short) are a class of non-coding, single stranded, small ribonucleic acid molecules with 19-23 nucleotides in length. They exist extensively within the cells of animals and plants and are highly conserved in evolution. MicroRNA inhibits the translation of corresponding proteins through recognizing 3' end non-translated sequence of a target messenger RNA (mRNA) which is not completely complementary thereto. As a powerful regulatory factor for mRNA, microRNA is closely related to physiological or pathological activities. It relates to life activities such as the individual development, tissue differentiation, apoptosis and energy metabolism of organisms; and it is also closely related to the occurrence and development of many diseases.

### Human milk microRNAs

It is demonstrated through the study of the present inventors that this extremely important factor -- microRNAs exists in human milk.

Also, it is found that the majority of microRNAs existing stably in human milk are related to the development of the immune system and nerves.

The microRNAs in human milk which are related to immunity include miR-181a, miR-223, miR-146a and the like, for instance, miR-181a is related to the development of B cells, the activation of T cells, and the development of the immune system. miR181a promotes the development of B cells and the selection, activation and sensitivity of CD4+T cells. The increase of expression of miR181 a in mature T cells can enhance the sensitivity of peptide antigen. miR-223 participates in defence through negatively regulating the proliferation and activation of neutrophils. miR-223 also positively regulates the differentiation of granulocytes and miR-223 negatively regulates inflammatory reaction. miR-146a participates in defence through negatively regulating congenital immunity. The expression of miR-146a is increased in alveolar epithelium and it negatively regulates IL-8 and RANTES which are induced by IL-1B, and thus activating and regulating the expression and secretion of normal T cells.

The microRNAs in human milk which are related to nerves include miR-140-3p, miR-107, miR-19a and the like, for instance, miR-140-3p is related to the development of fetal nerve stem cells. miR-107 directly targets the 3' non-coding region of CDK5R1 and affects the migration of nerve cells through regulating the expression of CDK5R1. The expression of miR-19a in neuroblastoma cells is upregulated, and it targets and inhibits estrogen receptor α (ESR1), and ESR1 is a transcription factor with expression thereof induced by a ligand and related to the differentiation of nerve cells.

Besides the microRNAs existing in human colostrum being related to the immune system and nerve system, there still are some microRNAs, such as miR-33a*, miR-326, and miR-488, which participate in other physiological/pathological activities. For example, miR-33 family members (hsa-miR-33a and hsa-miR-33b) regulate the metabolism of cholesterol and fatty acids. miR-33 family members also regulate the gene expression which participates in cell cycle regulation and cell proliferation. miR-33 inhibits the expression of cyclin-dependent kinase 6 (CDK6) and cyclin D1 (CCND1 protein), thus slowing down the processes for cell proliferation and cell cycle. miR-33a in pancreatic islet cells regulates the expression of ATP binding cassette transporter A1 (ABCA1), the accumulation of cholesterol and the secretion of insulin. miR-326 prevents and resists multidrug resistance during the chemotherapy of breast cancer through regulating the expression of multidrug resistance-associated protein 1 (MRP-1/ABCC1). miR-488 overexpresses in androgen-dependent prostate cancer cells and androgen-independent prostate cancer cells, downregulates the transcription activity of androgen receptors, and inhibits the generation of androgen receptor protein. miR-488 hampers the diffusion of prostate cancer cells and promotes the apoptosis of prostate cancer cells.

Therefore, novel guiding criteria can be provided for the formula of infant foods (such as milk product) if the varieties of microRNAs existing in breast milk and expression level thereof can be detected to form the expression profiles of the microRNAs of human milk or specific to human milk; and the scientific staging of infant foods can be guided if the varieties of microRNAs existing in the breast milk secreted by a mother in different periods (such as colostrum or mature breast milk) and expression level thereof can be detected to form the expression profiles of the microRNAs specific to human milk in different periods. In addition, the inventors can also selectively increase and/or decrease the contents of certain kinds of microRNAs in milk product according to the expression profiles of the microRNAs of human milk, so as to prepare foods, food supplements and novel nutraceuticals directed to special infants; and the method for making novel criteria for milk products can also be guided.

To this end, the inventors detect the varieties and contents of the microRNAs in human milk and screen and compare the detection results to establish the expression profiles of the microRNAs of human milk; and further detect the varieties and contents of the microRNAs in breast milk secreted by a mother in different periods (including colostrum and mature breast milk) and screen and compare the detection results to establish the expression profiles of the microRNAs specific to colostrum and/or mature breast milk. These technical progresses occur for the first time in this technical field.

### Detection

A series of highly efficient and stable methods for detecting human milk microRNAs have been selected by the inventors through a series of studies on the detection of human milk microRNAs and application thereof, said methods for detecting human milk microRNAs include (but not limited to): Solexa sequencing technique, RT-PCR, Real-time PCR, Northern Blotting, Western Blotting and the like.

Furthermore, the detection results for human milk microRNAs (including varieties and contents) showed that large amount of microRNAs related to immune system and nerve system exist in human milk, and human milk microRNAs may play a crucial role in immunity and nerve development of infants, thus forming a series of microRNA expression profiles of human milk or specific to human milk. In addition, the detection results of microRNAs (including varieties and contents) in the breast milk secreted by a mother in different periods (including colostrum and/or mature breast milk) obtained by the inventors showed that there are differences in the expression of microRNAs in human milk in different periods, therefore, the microRNAs in human milk in different periods are likely to have different effects on the growth and development of infants in different periods, thus forming a series of microRNA expression profiles specific to mature breast milk or colostrum.

### microRNA expression profiles

Said microRNA expression profiles can be the existing expression profiles of the microRNAs in a human mature breast milk sample or a human colostrum sample used for control, for instance, the expression profiles as shown in Table E;

**Table E**

| | | | | | | | | |
|---|---|---|---|---|---|---|---|---|
| let-7a | miR-100 | miR-1307 | miR-155 | miR-185 | miR-210 | miR-24 | miR-378 | miR-584 |
| let-7b | miR-101 | miR-141 | miR-16 | miR-186 | miR-212 | miR-25 | miR-425 | miR-652 |
| let-7c | miR-103 | miR-142-3p | miR-17 | miR-192 | miR-217 | miR-31 | miR-429 | miR-664 |
| let-7d | miR-107 | miR-142-5p | miR-181a | miR-195 | miR-218 | miR-328 | miR-451 | miR-708 |
| let-7e | miR-124 | miR-143 | miR-181b | miR-203 | miR-22 | miR-340 | miR-452 | miR-760 |
| let-7f | miR-125b | miR-146a | miR-182 | miR-204 | miR-221 | miR-345 | miR-484 | miR-874 |
| let-7i | miR-126 | miR-150 | miR-183 | miR-205 | miR-222 | miR-365 | miR-497 | miR-877 |
| miR-1 | miR-128 | miR-152 | miR-184 | miR-21 | miR-223 | miR-375 | miR-503 | miR-92a |
| | | | | | | miR-98 | miR-96 | miR-93 |

Said microRNA expression profiles can also be the microRNA expression profiles of the present invention. Said "microRNA expression profiles of the present invention" are preferably the following expression profiles:
1. microRNA expression profiles specific to human colostrum, as shown in Table A.

**Table A**

| No. | miR variety | No. | miR variety | No. | miR variety | No. | miR variety |
|---|---|---|---|---|---|---|---|
| 1 | miR-148a* | 25 | miR-130a | 49 | miR-185* | 73 | miR-424 |
| 2 | miR-19a | 26 | miR-132* | 50 | miR-195* | 74 | miR-450a |
| 3 | miR-19b | 27 | miR-1537 | 51 | miR-199b-5p | 75 | miR-450b-5p |
| 4 | miR-21* | 28 | miR-16-2* | 52 | miR-2115 | 76 | miR-502-3p |
| 5 | miR-27b | 29 | miR-17* | 53 | miR-2115* | 77 | miR-505 |
| 6 | miR-30e | 30 | miR-181d | 54 | miR-2355-3p | 78 | miR-548c-5p |
| 7 | miR-99a | 31 | miR-197 | 55 | miR-2355-5p | 79 | miR-548d-5p |
| 8 | miR-140-3p | 32 | miR-26b* | 56 | miR-27b* | 80 | miR-548o |
| 9 | miR-181a* | 33 | miR-27a* | 57 | miR-296-5p | 81 | miR-551a |
| 10 | miR-23a | 34 | miR-30d* | 58 | miR-29a* | 82 | miR-574-5p |
| 11 | miR-23b | 35 | miR-342-5p | 59 | miR-3158 | 83 | miR-576-3p |
| 12 | miR-27a | 36 | miR-423-5p | 60 | miR-3182 | 84 | miR-616* |
| 13 | miR-320a | 37 | miR-454* | 61 | miR-320b | 85 | miR-618 |
| 14 | miR-34a | 38 | miR-501-3p | 62 | miR-320c | 86 | miR-625* |
| 15 | let-7a* | 39 | miR-548i | 63 | miR-320d | 87 | miR-627 |
| 16 | let-7b* | 40 | miR-548k | 64 | miR-331-3p | 88 | miR-629* |
| 17 | let-7d* | 41 | miR-629 | 65 | miR-338-3p | 89 | miR-720 |
| 18 | let-7f-1* | 42 | miR-383 | 66 | miR-34a* | 90 | miR-766 |
| 19 | let-7f-2* | 43 | miR-10b | 67 | miR-34b* | 91 | miR-873 |
| 20 | let-7g* | 44 | miR-1275 | 68 | miR-34c-5p | 92 | miR-93* |
| 21 | miR-1246 | 45 | miR-1299 | 69 | miR-361-3p | 93 | miR-934 |
| 22 | miR-126* | 46 | miR-136* | 70 | miR-3909 | 94 | miR-944 |
| 23 | miR-1260 | 47 | miR-143* | 71 | miR-3913 | 95 | miR-145* |
| 24 | miR-1260b | 48 | miR-147b | 72 | miR-410 | 96 | miR-146a* |

microRNA expression profiles specific to human colostrum preferably include miRs numbered as 1-42 in Table A.
2. microRNA expression profiles specific to human mature breast milk are as shown in Table B;

**Table B**

| No. | miR variety | No. | miR variety | No. | miR variety | No. | miR variety |
|---|---|---|---|---|---|---|---|
| 1 | let-7g | 16 | miR-153 | 31 | miR-376a | 46 | miR-548v |
| 2 | miR-30b | 17 | miR-19b-1* | 32 | miR-411 | 47 | miR-552 |
| 3 | miR-200a* | 18 | miR-2110 | 33 | miR-4297 | 48 | miR-589* |
| 4 | miR-200c | 19 | miR-2116* | 34 | miR-488 | 49 | miR-641 |
| 5 | miR-18a | 20 | miR-2277-3p | 35 | miR-491-5p | 50 | miR-643 |
| 6 | miR-196b | 21 | miR-3065-3p | 36 | miR-514 | 51 | miR-95 |
| 7 | miR-200b* | 22 | miR-3074 | 37 | miR-516b | 52 | miR-3200-3p |
| 8 | miR-224 | 23 | miR-3115 | 38 | miR-517c | 53 | miR-339-5p |
| 9 | miR-511 | 24 | miR-3174 | 39 | miR-518e | 54 | miR-499-3p |
| 10 | miR-99a* | 25 | miR-3190 | 40 | miR-519a* | 55 | miR-512-3p |
| 11 | miR-3656 | 26 | miR-3200-5p | 41 | miR-519b-3p | 56 | miR-517a |
| 12 | miR-1228* | 27 | miR-326 | 42 | miR-520a-5p | 57 | miR-517b |
| 13 | miR-1249 | 28 | miR-3617 | 43 | miR-520f | 58 | miR-519a |
| 14 | miR-1276 | 29 | miR-3620 | 44 | miR-522 | 59 | miR-519c-3p |
| 15 | miR-1283 | 30 | miR-3654 | 45 | miR-524-3p | 60 | miR-671-3p |
| | | | | | | 61 | miR-937 |

More preferably, microRNA expression profiles specific to human mature breast milk are miRs numbered as 1-11 in Table B.
3. Expression profiles of common microRNAs both in human colostrum and mature breast milk are as shown in Table C;

**Table C**

| No. | miR variety | No. | miR variety | No. | miR variety | No. | miR variety |
|---|---|---|---|---|---|---|---|
| 1 | let-7a* | 87 | miR-190 | 173 | miR-3196 | 259 | miR-500b |
| 2 | let-7b* | 88 | miR-190b | 174 | miR-32 | 260 | miR-501-3p |
| 3 | let-7d* | 89 | miR-191 | 175 | miR-32* | 261 | miR-501-5p |
| 4 | let-7e* | 90 | miR-192* | 176 | miR-3200-3p | 262 | miR-502-3p |
| 5 | let-7f-1* | 91 | miR-193a-3p | 177 | miR-320a | 263 | miR-502-5p |
| 6 | let-7f-2* | 92 | miR-193a-5p | 178 | miR-320b | 264 | miR-504 |
| 7 | let-7g | 93 | miR-193b | 179 | miR-320c | 265 | miR-505 |
| 8 | let-7g* | 94 | miR-194 | 180 | miR-320d | 266 | miR-511 |
| 9 | let-7i* | 95 | miR-195* | 181 | miR-324-3p | 267 | miR-512-3p |
| 10 | miR-100* | 96 | miR-196a | 182 | miR-324-5p | 268 | miR-515-5p |
| 11 | miR-101* | 97 | miR-196a* | 183 | miR-330-3p | 269 | miR-517a |
| 12 | miR-103-2* | 98 | miR-196b | 184 | miR-330-5p | 270 | miR-517b |
| 13 | miR-106a | 99 | miR-197 | 185 | miR-331-3p | 271 | miR-519a |
| 14 | miR-106b | 100 | miR-199a-3p | 186 | miR-331-5p | 272 | miR-519c-3p |
| 15 | miR-106b* | 101 | miR-199b-3p | 187 | miR-335 | 273 | miR-520a-3p |
| 16 | miR-10a | 102 | miR-199b-5p | 188 | miR-335* | 274 | miR-532-3p |
| 17 | miR-10a* | 103 | miR-19a | 189 | miR-338-3p | 275 | miR-532-5p |
| 18 | miR-10b | 104 | miR-19b | 190 | miR-339-3p | 276 | miR-542-3p |
| 19 | miR-1246 | 105 | miR-200a | 191 | miR-339-5p | 277 | miR-548c-5p |
| 20 | miR-1251 | 106 | miR-200a* | 192 | miR-33a | 278 | miR-548d-5p |
| 21 | miR-125a-3p | 107 | miR-200b | 193 | miR-33a* | 279 | miR-548e |
| 22 | miR-125a-5p | 108 | miR-200b* | 194 | miR-33b | 280 | miR-548h |
| 23 | miR-125b-1* | 109 | miR-200c | 195 | miR-33b* | 281 | miR-548i |
| 24 | miR-125b-2* | 110 | miR-200c* | 196 | miR-340* | 282 | miR-548j |
| 25 | miR-126* | 111 | miR-20a | 197 | miR-342-3p | 283 | miR-548k |
| 26 | miR-1260 | 112 | miR-20a* | 198 | miR-342-5p | 284 | miR-548n |
| 27 | miR-1260b | 113 | miR-20b | 199 | miR-34a | 285 | miR-548o |
| 28 | miR-1262 | 114 | miR-21* | 200 | miR-34a* | 286 | miR-548p |
| 29 | miR-1266 | 115 | miR-2114 | 201 | miR-34b* | 287 | miR-548y |
| 30 | miR-1271 | 116 | miR-2114* | 202 | miR-34c-5p | 288 | miR-548z |
| 31 | miR-127-3p | 117 | miR-2115 | 203 | miR-3605-5p | 289 | miR-550a* |
| 32 | miR-1274b | 118 | miR-2115* | 204 | miR-3613-5p | 290 | miR-551a |
| 33 | miR-1275 | 119 | miR-2116 | 205 | miR-361-3p | 291 | miR-574-3p |
| 34 | miR-1280 | 120 | miR-215 | 206 | miR-3614-5p | 292 | miR-574-5p |
| 35 | miR-1287 | 121 | miR-219-1-3p | 207 | miR-3615 | 293 | miR-576-3p |
| 36 | miR-1294 | 122 | miR-219-5p | 208 | miR-361-5p | 294 | miR-576-5p |
| 37 | miR-1296 | 123 | miR-22* | 209 | miR-362-3p | 295 | miR-577 |
| 38 | miR-1299 | 124 | miR-221* | 210 | miR-362-5p | 296 | miR-579 |
| 39 | miR-1301 | 125 | miR-223* | 211 | miR-363 | 297 | miR-580 |
| 40 | miR-1303 | 126 | miR-224 | 212 | miR-365* | 298 | miR-582-3p |
| 41 | miR-130a | 127 | miR-224* | 213 | miR-3656 | 299 | miR-582-5p |
| 42 | miR-130b | 128 | miR-2277-5p | 214 | miR-3664 | 300 | miR-589 |
| 43 | miR-130b* | 129 | miR-2355-3p | 215 | miR-3677 | 301 | miR-590-3p |
| 44 | miR-132 | 130 | miR-2355-5p | 216 | miR-3688 | 302 | miR-590-5p |
| 45 | miR-132* | 131 | miR-23a | 217 | miR-369-3p | 303 | miR-597 |
| 46 | miR-135a | 132 | miR-23b | 218 | miR-374a | 304 | miR-598 |
| 47 | miR-135b | 133 | miR-24-2* | 219 | miR-374a* | 305 | miR-616* |
| 48 | miR-136* | 134 | miR-26a | 220 | miR-374b | 306 | miR-618 |
| 49 | miR-140-3p | 135 | miR-26a-1* | 221 | miR-374b* | 307 | miR-624* |
| 50 | miR-140-5p | 136 | miR-26b | 222 | miR-377 | 308 | miR-625 |
| 51 | miR-141* | 137 | miR-26b* | 223 | miR-378* | 309 | miR-625* |
| 52 | miR-143* | 138 | miR-27a | 224 | miR-381 | 310 | miR-627 |
| 53 | miR-144* | 139 | miR-27a* | 225 | miR-382 | 311 | miR-628-3p |
| 54 | miR-145 | 140 | miR-27b | 226 | miR-383 | 312 | miR-628-5p |
| 55 | miR-1468 | 141 | miR-27b* | 227 | miR-3909 | 313 | miR-629 |
| 56 | miR-146b-3p | 142 | miR-28-3p | 228 | miR-3912 | 314 | miR-629* |
| 57 | miR-146b-5p | 143 | miR-28-5p | 229 | miR-3913 | 315 | miR-642a |
| 58 | miR-147b | 144 | miR-296-5p | 230 | miR-3919 | 316 | miR-651 |
| 59 | miR-148a | 145 | miR-29a | 231 | miR-3920 | 317 | miR-660 |
| 60 | miR-148a* | 146 | miR-29a* | 232 | miR-3942 | 318 | miR-671-3p |
| 61 | miR-148b | 147 | miR-29b | 233 | miR-409-3p | 319 | miR-671-5p |
| 62 | miR-148b* | 148 | miR-29b-2* | 234 | miR-410 | 320 | miR-7 |
| 63 | miR-151-3p | 149 | miR-29c | 235 | miR-421 | 321 | miR-708* |
| 64 | miR-151-5p | 150 | miR-29c* | 236 | miR-422a | 322 | miR-7-1* |
| 65 | miR-1537 | 151 | miR-301a | 237 | miR-423-3p | 323 | miR-720 |
| 66 | miR-155* | 152 | miR-301b | 238 | miR-423-5p | 324 | miR-744 |
| 67 | miR-15a | 153 | miR-30a | 239 | miR-424 | 325 | miR-744* |
| 68 | miR-15a* | 154 | miR-30a* | 240 | miR-425* | 326 | miR-766 |
| 69 | miR-15b | 155 | miR-30b | 241 | miR-4284 | 327 | miR-769-3p |
| 70 | miR-15b* | 156 | miR-30b* | 242 | miR-4286 | 328 | miR-769-5p |
| 71 | miR-16-1* | 157 | miR-30c | 243 | miR-4326 | 329 | miR-873 |
| 72 | miR-16-2* | 158 | miR-30c-1* | 244 | miR-449a | 330 | miR-885-5p |
| 73 | miR-17* | 159 | miR-30c-2* | 245 | miR-449b | 331 | miR-889 |
| 74 | miR-181a* | 160 | miR-30d | 246 | miR-450a | 332 | miR-891a |
| 75 | miR-181a-2* | 161 | miR-30d* | 247 | miR-450b-5p | 333 | miR-9 |
| 76 | miR-181c | 162 | miR-30e | 248 | miR-454 | 334 | miR-92a-1* |
| 77 | miR-181c* | 163 | miR-30e* | 249 | miR-454* | 335 | miR-92b |
| 78 | miR-181d | 164 | miR-3116 | 250 | miR-455-3p | 336 | miR-93* |
| 79 | miR-182* | 165 | miR-3127 | 251 | miR-455-5p | 337 | miR-934 |
| 80 | miR-183* | 166 | miR-3130-3p | 252 | miR-486-5p | 338 | miR-937 |
| 81 | miR-185* | 167 | miR-3130-5p | 253 | miR-489 | 339 | miR-941 |
| 82 | miR-187 | 168 | miR-3133 | 254 | miR-493 | 340 | miR-944 |
| 83 | miR-188-3p | 169 | miR-3144-3p | 255 | miR-499-3p | 341 | miR-99a |
| 84 | miR-188-5p | 170 | miR-3158 | 256 | miR-499-5p | 342 | miR-99a* |
| 85 | miR-18a | 171 | miR-3182 | 257 | miR-500a | 343 | miR-99b |
| 86 | miR-18a* | 172 | miR-3194 | 258 | miR-500a* | 344 | miR-99b* |

4. Expression profiles of microRNAs not existing in cow milk but existing in human colostrum or mature breast milk are as shown in Table D.

**Table D**

| No. | miR variety | No. | miR variety | No. | miR variety | No. | miR variety |
|---|---|---|---|---|---|---|---|
| 1 | let-7a* | 61 | miR-200c* | 121 | miR-3664 | 181 | miR-589 |
| 2 | let-7b* | 62 | miR-20a* | 122 | miR-3677 | 182 | miR-590-3p |
| 3 | let-7d* | 63 | miR-20b | 123 | miR-3688 | 183 | miR-590-5p |
| 4 | let-7e* | 64 | miR-21* | 124 | miR-374a* | 184 | miR-597 |
| 5 | let-7f-1* | 65 | miR-2114 | 125 | miR-374b* | 185 | miR-598 |
| 6 | let-7f-2* | 66 | miR-2114* | 126 | miR-377 | 186 | miR-616* |
| 7 | let-7g* | 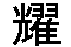 7 | miR-2115 | 127 | miR-378* | 187 | miR-618 |
| 8 | let-7i* | 68 | miR-2115* | 128 | miR-3909 | 188 | miR-624* |
| 9 | miR-100* | 69 | miR-2116 | 129 | miR-3912 | 189 | miR-625 |
| 10 | miR-101* | 70 | miR-22* | 130 | miR-3913 | 190 | miR-625* |
| 11 | miR-103-2* | 71 | miR-221* | 131 | miR-3919 | 191 | miR-627 |
| 12 | miR-106b* | 72 | miR-223* | 132 | miR-3920 | 192 | miR-629 |
| 13 | miR-10a* | 73 | miR-224* | 133 | miR-3942 | 193 | miR-629* |
| 14 | miR-1251 | 74 | miR-2277-5p | 134 | miR-409-3p | 194 | miR-642a |
| 15 | miR-125b-1* | 75 | miR-2355-3p | 135 | miR-410 | 195 | miR-651 |
| 16 | miR-125b-2* | 76 | miR-2355-5p | 136 | miR-422a | 196 | miR-708* |
| 17 | miR-126* | 77 | miR-24-2* | 137 | miR-425* | 197 | miR-7-1* |
| 18 | miR-1260b | 78 | miR-26a-1* | 138 | miR-4284 | 198 | miR-744* |
| 19 | miR-1262 | 79 | miR-26b* | 139 | miR-4286 | 199 | miR-766 |
| 20 | miR-1266 | 80 | miR-27a* | 140 | miR-4326 | 200 | miR-769-3p |
| 21 | miR-1271 | 81 | miR-27b* | 141 | miR-449b | 201 | miR-769-5p |
| 22 | miR-1275 | 82 | miR-28-3p | 142 | miR-454 | 202 | miR-873 |
| 23 | miR-1280 | 83 | miR-296-5p | 143 | miR-454* | 203 | miR-889 |
| 24 | miR-1287 | 84 | miR-29a* | 144 | miR-489 | 204 | miR-891a |
| 25 | miR-1294 | 85 | miR-29b-2* | 145 | miR-493 | 205 | miR-92a-1* |
| 26 | miR-1299 | 86 | miR-29c* | 146 | miR-499-3p | 206 | miR-93* |
| 27 | miR-1301 | 87 | miR-301b | 147 | miR-500a | 207 | miR-934 |
| 28 | miR-1303 | 88 | miR-30a* | 148 | miR-500a* | 208 | miR-937 |
| 29 | miR-130b* | 89 | miR-30b* | 149 | miR-500b | 209 | miR-941 |
| 30 | miR-132* | 90 | miR-30c-1* | 150 | miR-501-5p | 210 | miR-944 |
| 31 | miR-135b | 91 | miR-30c-2* | 151 | miR-502-5p | 211 | miR-99a* |
| 32 | miR-136* | 92 | miR-30d* | 152 | miR-504 | 212 | miR-99b* |
| 33 | miR-141* | 93 | miR-30e* | 153 | miR-511 | 213 | miR-10b |
| 34 | miR-143* | 94 | miR-3116 | 154 | miR-512-3p | 214 | miR-1260 |
| 35 | miR-144* | 95 | miR-3127 | 155 | miR-515-5p | 215 | miR-140-5p |
| 36 | miR-1468 | 96 | miR-3130-3p | 156 | miR-517a | 216 | miR-147b |
| 37 | miR-146b-3p | 97 | miR-3130-5p | 157 | miR-517b | 217 | miR-181c |
| 38 | miR-148a* | 98 | miR-3133 | 158 | miR-519a | 218 | miR-187 |
| 39 | miR-148b* | 99 | miR-3144-3p | 159 | miR-519c-3p | 219 | miR-188-3p |
| 40 | miR-1537 | 100 | miR-3158 | 160 | miR-520a-3p | 220 | miR-188-5p |
| 41 | miR-155* | 101 | miR-3182 | 161 | miR-548c-5p | 221 | miR-18a |
| 42 | miR-15a* | 102 | miR-3194 | 162 | miR-548d-5p | 222 | miR-190 |
| 43 | miR-15b* | 103 | miR-3196 | 163 | miR-548e | 223 | miR-190b |
| 44 | miR-16-1* | 104 | miR-32 | 164 | miR-548h | 224 | miR-19a |
| 45 | miR-16-2* | 105 | miR-32* | 165 | miR-548i | 225 | miR-219-1-3p |
| 46 | miR-17* | 106 | miR-3200-3p | 166 | miR-548j | 226 | miR-219-5p |
| 47 | miR-181a* | 107 | miR-335* | 167 | miR-548k | 227 | miR-301a |
| 48 | miR-181a-2* | 108 | miR-339-3p | 168 | miR-548n | 228 | miR-330-5p |
| 49 | miR-181c* | 109 | miR-33a* | 169 | miR-548o | 229 | miR-338-3p |
| 50 | miR-182* | 110 | miR-33b* | 170 | miR-548p | 230 | miR-34c-5p |
| 51 | miR-183* | 111 | miR-340* | 171 | miR-548y | 231 | miR-381 |
| 52 | miR-185* | 112 | miR-342-5p | 172 | miR-548z | 232 | miR-383 |
| 53 | miR-18a* | 113 | miR-34a* | 173 | miR-550a* | 233 | miR-424 |
| 54 | miR-192* | 114 | miR-34b* | 174 | miR-551a | 234 | miR-449a |
| 55 | miR-193b | 115 | miR-3605-5p | 175 | miR-576-3p | 235 | miR-450a |
| 56 | miR-195* | 116 | miR-3613-5p | 176 | miR-576-5p | 236 | miR-455-5p |
| 57 | miR-196a* | 117 | miR-3614-5p | 177 | miR-577 | 237 | miR-501-3p |
| 58 | miR-199b-5p | 118 | miR-3615 | 178 | miR-579 | 238 | miR-505 |
| 59 | miR-200a* | 119 | miR-365* | 179 | miR-580 | 239 | miR-542-3p |
| 60 | miR-200b* | 120 | miR-3656 | 180 | miR-582-3p | 240 | miR-582-5p |
| | | | | | | 241 | miR-9 |

It should be understood that in terms of Table A, Table B, Table C and Table D, the term "microRNAs listed in Tables" refers to one or more kinds of microRNAs listed in the Tables.

### microRNA set

As used herein, " the microRNAs of the present invention" refers to one or more kinds of microRNAs selected from the microRNA expression profiles of the present invention.

Preferably, the present invention provides an isolated microRNA set, said microRNA set is composed of two or more kinds (for example, 2-500 kinds, preferably 5-300 kinds, and more preferably 5-100 kinds) of microRNAs selected from the group comprising: (a) microRNAs specific to human colostrum and listed in Table A; (b) microRNAs specific to human mature breast milk and listed in Table B; (c) common microRNAs existing both in human colostrum and mature breast milk and listed in Table C; (d) microRNAs not existing in cow milk but existing in human colostrum or human mature breast milk, and listed in Table D.

Said microRNA set is used as an effective ingredient. It can be used for preparing artificial milk products, foods or food supplements, more preferably, the expression profiles of the microRNAs in said artificial milk products or food supplements are approximate or identical to the expression profiles of the microRNAs in a normal human colostrum sample or a normal human mature breast milk sample.

### Applications

The microRNAs of the present invention are microRNAs related to the immune system and nerve system and the like, and play a crucial role in immunity and nerve development of infants. Therefore, the microRNAs of the present invention or the microRNA expression profiles of the present invention have multiple aspects of uses, preferably as follows:

1. They can be used for guiding formulas of infant foods (such as milk products) or food supplements

Preferably, the present invention provides an artificial milk product which contains one or more kinds of microRNAs which are exogenously added and selected from the group comprising: (a) microRNAs specific to human colostrum and listed in Table A; (b) microRNAs specific to human mature breast milk and listed in Table B; (c) common microRNAs existing both in human colostrum and mature breast milk and listed in Table C; (d) microRNAs not existing in cow milk but existing in human colostrum or human mature breast milk, and listed in Table D.

In another preferred example, said milk product also contains one or more kinds of microRNAs which are added exogenously and selected from the group comprising: (e) the microRNAs listed in Table E. Said microRNAs listed in Table E are known microRNAs which have been reported in documents.

Said artificial milk product can be a milk product applicable to infants of 0-24 months old; or a milk product applicable to population above 2 years old.

Preferably, said milk product includes a colostrum product and a mature breast milk product. Said milk product can also preferably include milk powder, liquid milk, cheese, yogurt and the like.

Said milk product can have one or more of the following characteristics: (a1) two or more kinds (for example, 2-500 kinds, preferably 3-300 kinds, and more preferably 3-200 kinds) of said exogenously added microRNAs are derived from Table A; (a2) the varieties and contents of said exogenously added microRNAs are approximate or identical to the microRNA expression profiles of human colostrum; (b1) two or more kinds (for example, 2-500 kinds, preferably 3-300 kinds, and more preferably 3-200 kinds) of said exogenously added microRNAs are derived from Table B; (b2) the varieties and contents of said exogenously added microRNAs are approximate or identical to the microRNA expression profiles of human mature breast milk; (c1) two or more kinds (for example, 2-500 kinds, preferably 3-300 kinds, and more preferably 3-200 kinds) of said exogenously added microRNAs are derived from Table C; (d1) two or more kinds (for example, 2-500 kinds, preferably 3-300 kinds, and more preferably 3-200 kinds) of said exogenously added microRNAs are derived from Table D.

Preferably, the present invention provides an artificial food supplement which contains a carrier acceptable in bromatology and one or more kinds of microRNAs selected from the group comprising: (a) microRNAs specific to human colostrum and listed in Table A; (b) microRNAs specific to human mature breast milk and listed in Table B; (c) common microRNAs existing both in human colostrum and mature breast milk and listed in Table C; (d) microRNAs not existing in cow milk but existing in human colostrum or human mature breast milk, and listed in Table D.

In another preferred example, said food supplement also contains one or more kinds of microRNAs selected from the group comprising: (e) the microRNAs listed in Table E as described above.

Said food supplement can have one or more of the following characteristics: (a1) two or more kinds (for example, 2-500 kinds, preferably 3-300 kinds, and more preferably 3-200 kinds) of said exogenously added microRNAs are derived from Table A; (a2) the varieties and contents of said exogenously added microRNAs are approximate or identical to the microRNA expression profiles of human colostrum; (b1) two or more kinds (for example, 2-500 kinds, preferably 3-300 kinds, and more preferably 3-200 kinds) of said exogenously added microRNAs are derived from Table B; (b2) the varieties and contents of said exogenously added microRNAs are approximate or identical to the microRNA expression profiles of human mature breast milk; (c1) two or more kinds (for example, 2-500 kinds, preferably 3-300 kinds, and more preferably 3-200 kinds) of said exogenously added microRNAs are derived from Table C; (d1) two or more kinds (for example, 2-500 kinds, preferably 3-300 kinds, and more preferably 3-200 kinds) of said exogenously added microRNAs are derived from Table D.

2. They can be used for guiding the scientific staging of infant foods (including milk products)

Preferably, the present invention provides a method of identifying a milk product, said method comprises the steps of:
(1) Provides a milk product (such as milk powder) sample (preferably including cow milk or a milk product derived therefrom, goat's milk or a milk product derived therefrom, human milk and the like);
(2) detecting the varieties of the microRNAs in said milk product sample and the contents thereof;
(3) comparing the varieties of the microRNAs and the contents thereof detected in step (2) with the expression profiles (i.e., the varieties of microRNAs and the contents thereof) of the microRNAs in a colostrum sample and a mature breast milk sample used as controls;
wherein, said milk product is identified as a colostrum product if the detected varieties of the microRNAs and the contents thereof are approximate or identical to the expression profiles of the microRNAs of the colostrum sample; and said milk product is identified as a mature breast milk product if the detected varieties of the microRNAs and the contents thereof are approximate or identical to the expression profiles of the microRNAs of the mature breast milk sample.

In another preferred example, the expression profiles of the microRNAs in a mature breast milk sample or a colostrum sample used as control are obtained through detecting the varieties of microRNAs in mature breast milk or colostrum and the contents thereof.

Said expression profiles of the microRNAs in the mature breast milk sample or the colostrum sample used as control can also comprise the microRNAs of the present invention, and can also comprise the microRNAs known in prior art (for example, the microRNA listed in said Table E). Preferably, the expression profiles of the microRNAs in said colostrum sample used as control comprises two or more kinds of microRNAs listed in Table A and Table C; and the expression profiles of the microRNAs in said mature breast milk sample used as control comprises the microRNAs listed in Table B and Table C.

3. The contents of certain kinds of microRNAs in infant foods (such as milk products) can be selectively increased and/or decreased according to the expression profiles of the microRNAs of human milk, so as to prepare foods (such as milk products), food supplements, novel nutraceuticals and the like directed to special infants.

Preferably, the present invention provides a method of manufacturing a milk product, said method comprises the steps of:
(1) providing a milk product material (including cow milk, goat's milk, or a combination thereof);
(2) detecting the varieties of the microRNAs in said milk product material and the contents thereof to obtain a first microRNA expression profile;
(3) comparing the varieties of the microRNAs and the contents thereof detected in step (2) with he expression profiles of the microRNAs in a colostrum sample or a mature breast milk sample used as control;
(4) Based on the comparison results, adding one or more kinds of microRNAs to said milk product material and/or removing one or more kinds of microRNAs from said milk product material to give the milk product, wherein the expression profiles of the microRNAs in said milk product are more approximate or identical to the expression profiles of the microRNAs in the colostrum sample or the mature breast milk sample used as control.

In another preferred example, the expression profiles of the microRNAs in the mature breast milk sample or the colostrum sample used as control, the method for obtaining the expression profiles and the microRNAs contained therein are as mentioned before.

Preferably, the present invention also provides a method of manufacturing a milk product, said method comprises the steps of:
(1) providing a milk product material;
(2) adding one or more kinds of microRNAs to said milk product material and/or removing one or more kinds of microRNAs from said milk product material to give a milk product, wherein expression profile of the microRNAs in said milk product is more approximate or identical to the expression profile of the microRNAs in the colostrum or the mature breast milk.

In another preferred example, said colostrum or mature breast milk is human colostrum or human mature breast milk.

In this case, said one or more kinds of microRNAs can comprise the microRNAs of the present invention, and can also comprise the microRNAs known in prior art (for example, the microRNA listed in said Table E). More preferably, they comprise the microRNAs of the present invention: one or more kinds of microRNAs listed in Table A, Table B, Table C and/or Table D.

Preferably, the present invention also provides a method of manufacturing a food supplement, said method comprises the steps of:
(1) detecting the varieties of the microRNAs in the breast milk of a certain subject and the contents thereof to obtain a first microRNA expression profile of the individual;
(2) comparing the detected varieties of the microRNAs of the first expression profile and the contents thereof with the expression profiles of the microRNAs in a normal colostrum sample or a normal mature breast milk sample used as control, so as to determine the microRNAs which are lack in the first expression profile or the microRNAs which are insufficient and needed to be supplemented;
(3) preparing the food supplement, wherein said food supplement contains the microRNAs as determined in the foregoing step, which are lack in the first expression profile or are insufficient and needed to be supplemented.

In another preferred example, in the preparation step, included is mixing a carrier acceptable in bromatology with one or more artificially synthesized microRNAs so as to form a food supplement.

In another preferred example, the expression profiles of the microRNAs in a normal mature breast milk sample or a normal colostrum sample used as control are obtained through detecting the varieties of microRNAs in the normal mature breast milk or the normal colostrum and the contents thereof.

In this case, said normal mature breast milk sample or normal colostrum sample indicates that said mature breast milk or colostrum contains microRNAs adequate (including varieties and contents) for the growth and development of infants.

The main advantages of the present invention include:
1. The present invention provides a novel microRNA set, the microRNAs in said set can be used for preparing artificial milk products, foods or food supplements and the like.
2. The present invention establishes a series of microRNA expression profiles of human milk, including the microRNA expression profiles of human milk (broadly or specifically), the microRNA expression profiles specific to human colostrum, or the microRNA expression profiles specific to human mature breast milk and the like, as well as the uses of said microRNA expression profiles, said microRNA expression profiles can be used for guiding the preparation of artificial milk products, foods or food supplements; and can also be used for identifying a milk product as a mature breast milk product or a colostrum product.

The present invention is further illustrated in connection with particular embodiments as follows. It should be understood that these embodiments are merely illustrative of the invention and are not intended to limit the scope of the present invention. Where the specific conditions for an experimental method are not specified in the following examples, conventional conditions are generally followed, such as the conditions described in Sambrook et al., *Molecular Cloning: A Laboratory Manual* (New York: Cold Spring Harbor Laboratory Press, 1989), or the conditions recommended by the manufacturer are followed. All percentages and parts are by weight unless otherwise indicated. GFP represents green fluorescent protein.

### Example 1: microRNAs stably existing in human milk

A large amount of microRNAs stably existing in human milk are detected using Solexa sequencing technique.

The specific steps include:
(1) collecting a human milk sample;
(2) extracting total RNA from the sample with a Trizol reagent;
(3) conducting PAGE electrophoresis to recover RNA molecules of 17-27nt;
(4) enzyme-linking adaptor primers to the 3' and 5' ends of the small RNA molecules respectively;
(5) conducting cluster generation directly using purified DNA, and conducting sequencing analysis utilizing Illumina Genome Analyzer.

See the particular results in Table 1. Table 1 is the sequencing results of microRNAs in human milk. It can be seen from the sequencing results that a large amount of microRNAs stably exist in human milk.

**Table 1 microRNAs contained in human milk**

| | | | | | | |
|---|---|---|---|---|---|---|
| let-7a* | miR-141* | miR-199b-3p | miR-301 a | miR-34b* | miR-455-5p | miR-590-3p |
| let-7b* | miR-143* | miR-199b-5p | miR-301b | miR-34c-5p | miR-486-5p | miR-590-5p |
| let-7d* | miR-144* | miR-19a | miR-30a | miR-3605-5p | miR-489 | miR-597 |
| let-7e* | miR-145 | miR-19b | miR-30a* | miR-3613-5p | miR-493 | miR-598 |
| let-7f-1* | miR-1468 | miR-200a | miR-30b | miR-361-3p | miR-499-3p | miR-616* |
| let-7f-2* | miR-146b-3p | miR-200a* | miR-30b* | miR-3614-5p | miR-499-5p | miR-618 |
| let-7g | miR-146b-5p | miR-200b | miR-30c | miR-3615 | miR-500a | miR-624* |
| let-7g* | miR-147b | miR-200b* | miR-30c-1* | miR-361-5p | miR-500a* | miR-625 |
| let-7i* | miR-148a | miR-200c | miR-30c-2* | miR-362-3p | miR-500b | miR-625* |
| miR-100* | miR-148a* | miR-200c* | miR-30d | miR-362-5p | miR-501-3p | miR-627 |
| miR-101* | miR-148b | miR-20a | miR-30d* | miR-363 | miR-501-5p | miR-628-3p |
| miR-103-2* | miR-148b* | miR-20a* | miR-30e | miR-365* | miR-502-3p | miR-628-5p |
| miR-106a | miR-151-3p | miR-20b | miR-30e* | miR-3656 | miR-502-5p | miR-629 |
| miR-106b | miR-151-5p | miR-21* | miR-3116 | miR-3664 | miR-504 | miR-629* |
| miR-106b* | miR-1537 | miR-2114 | miR-3127 | miR-3677 | miR-505 | miR-642a |
| miR-10a | miR-155* | miR-2114* | miR-3130-3p | miR-3688 | miR-511 | miR-651 |
| miR-10a* | miR-15a | miR-2115 | miR-3130-5p | miR-369-3p | miR-512-3p | miR-660 |
| miR-10b | miR-15a* | miR-2115* | miR-3133 | miR-374a | miR-515-5p | miR-671-3p |
| miR-1246 | miR-15b | miR-2116 | miR-3144-3p | miR-374a* | miR-517a | miR-671-5p |
| miR-1251 | miR-15b* | miR-215 | miR-3158 | miR-374b | miR-517b | miR-7 |
| miR-125a-3p | miR-16-1* | miR-219-1-3p | miR-3182 | miR-374b* | miR-519a | miR-708* |
| miR-125a-5p | miR-16-2* | miR-219-5p | miR-3194 | miR-377 | miR-519c-3p | miR-7-1* |
| miR-125b-1* | miR-17* | miR-22* | miR-3196 | miR-378* | miR-520a-3p | miR-720 |
| miR-125b-2* | miR-181a* | miR-221* | miR-32 | miR-381 | miR-532-3p | miR-744 |
| miR-126* | miR-181a-2* | miR-223* | miR-32* | miR-382 | miR-532-5p | miR-744* |
| miR-1260 | miR-181c | miR-224 | miR-3200-3p | miR-383 | miR-542-3p | miR-766 |
| miR-1260b | miR-181c* | miR-224* | miR-320a | miR-3909 | miR-548c-5p | miR-769-3p |
| miR-1262 | miR-181 d | miR-2277-5p | miR-320b | miR-3912 | miR-548d-5p | miR-769-5p |
| miR-1266 | miR-182* | miR-2355-3p | miR-320c | miR-3913 | miR-548e | miR-873 |
| miR-1271 | miR-183* | miR-2355-5p | miR-320d | miR-3919 | miR-548h | miR-885-5p |
| miR-127-3p | miR-185* | miR-23a | miR-324-3p | miR-3920 | miR-548i | miR-889 |
| miR-1274b | miR-187 | miR-23b | miR-324-5p | miR-3942 | miR-548j | miR-891a |
| miR-1275 | miR-188-3p | miR-24-2* | miR-330-3p | miR-409-3p | miR-548k | miR-9 |
| miR-1280 | miR-188-5p | miR-26a | miR-330-5p | miR-410 | miR-548n | miR-92a-1* |
| miR-1287 | miR-18a | miR-26a-1* | miR-331-3p | miR-421 | miR-548o | miR-92b |
| miR-1294 | miR-18a* | miR-26b | miR-331-5p | miR-422a | miR-548p | miR-93* |
| miR-1296 | miR-190 | miR-26b* | miR-335 | miR-423-3p | miR-548y | miR-934 |
| miR-1299 | miR-190b | miR-27a | miR-335* | miR-423-5p | miR-548z | miR-937 |
| miR-1301 | miR-191 | miR-27a* | miR-338-3p | miR-424 | miR-550a* | miR-941 |
| miR-1303 | miR-192* | miR-27b | miR-339-3p | miR-425* | miR-551a | miR-944 |
| miR-130a | miR-193a-3p | miR-27b* | miR-339-5p | miR-4284 | miR-574-3p | miR-99a |
| miR-130b | miR-193a-5p | miR-28-3p | miR-33a | miR-4286 | miR-574-5p | miR-99a* |
| miR-130b* | miR-193b | miR-28-5p | miR-33a* | miR-4326 | miR-576-3p | miR-99b |
| miR-132 | miR-194 | miR-296-5p | miR-33b | miR-449a | miR-576-5p | miR-99b* |
| miR-132* | miR-195* | miR-29a | miR-33b* | miR-449b | miR-577 | |
| miR-135a | miR-196a | miR-29a* | miR-340* | miR-450a | miR-579 | |
| miR-135b | miR-196a* | miR-29b | miR-342-3p | miR-450b-5p | miR-580 | |
| miR-136* | miR-196b | miR-29b-2* | miR-342-5p | miR-454 | miR-582-3p | |
| miR-140-3p | miR-197 | miR-29c | miR-34a | miR-454* | miR-582-5p | |
| miR-140-5p | miR-199a-3p | miR-29c* | miR-34a* | miR-455-3p | miR-589 | |

### Example 2: analysis of expression of microRNAs in human mature breast milk and colostrum

An unified standard for detecting human milk microRNAs is established in this example in combination with the Solexa sequencing results of the microRNAs in human mature breast milk and colostrum.

The specific steps for Solexa sequencing are as described in example 1 with the exception that the collected samples are human mature breast milk and colostrum, respectively.

The detection results are classified as follows using the range of copy numbers in colostrum and mature breast milk (in other examples, the method for classifying the detection results is the same as this method).

| Copy numbers | Content classification |
|---|---|
| 0 | non-existence/undetectable (represented by "UD") |
| 1-10 | low |
| 11-100 | relatively low |
| 101-1,000 | moderate |
| 1,001-10,000 | relatively high |
| 10,001-100,000 | high |

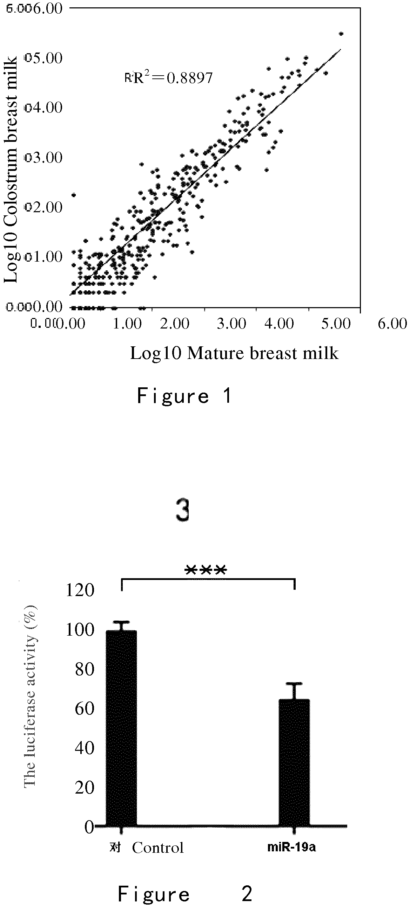

See the particular results in Table 2 and Figure 1. Table 2 is a table for the analysis of the differential expression of microRNAs in human mature breast milk and colostrum It can be seen from the results that the majority of microRNAs can be detected in both human mature breast milk and colostrum, and only a small part of microRNAs are specifically expressed in human mature breast milk or colostrum. There is no great difference with respect to the varieties of the microRNAs expressed in human mature breast milk compared with colostrum, but there is difference in terms of the expression amount of many microRNAs (such as let-7a*, let-7b*, let-7d* and the like) between the human mature breast milk and colostrum.

**Table 2 microRNAs contained in human mature breast milk or colostrum**

| No. | miR variety | miR content (human mature breast milk) | miR content (human colostrum ) | No. | miR variety | miR content (human mature breast milk) | miR content (human colostrum) |
|---|---|---|---|---|---|---|---|
| 1 | let-7a* | relatively low | moderate | 173 | miR-3196 | low | low |
| 2 | let-7b* | relatively low | moderate | 174 | miR-32 | relatively low | relatively low |
| 3 | let-7d* | relatively low | moderate | 175 | miR-32* | low | low |
| 4 | let-7e* | low | low | 176 | miR-3200-3 p | relatively low | low |
| 5 | let-7f-1* | relatively low | moderate | 177 | miR-320a | moderate | relatively high |
| 6 | let-7f-2* | relatively low | moderate | 178 | miR-320b | low | relatively low |
| 7 | let-7g | high | relatively high | 179 | miR-320c | low | relatively low |
| 8 | let-7g* | relatively low | moderate | 180 | miR-320d | low | relatively low |
| 9 | let-7i* | relatively low | relatively low | 181 | miR-324-3p | relatively low | relatively low |
| 10 | miR-100* | low | low | 182 | miR-324-5p | low | low |
| 11 | miR-101* | low | low | 183 | miR-330-3p | low | low |
| 12 | miR-103-2* | low | low | 184 | miR-330-5p | low | low |
| 13 | miR-106a | low | low | 185 | miR-331-3p | low | relatively low |
| 14 | miR-106b | moderate | moderate | 186 | miR-331-5p | low | low |
| 15 | miR-106b* | relatively high | relatively high | 187 | miR-335 | moderate | moderate |
| 16 | miR-10a | relatively high | relatively high | 188 | miR-335* | relatively high | relatively high |
| 17 | miR-10a* | low | low | 189 | miR-338-3p | low | relatively low |
| 18 | miR-10b | low | relatively low | 190 | miR-339-3p | low | low |
| 19 | miR-1246 | relatively low | moderate | 191 | miR-339-5p | relatively low | low |
| 20 | miR-1251 | relatively low | relatively low | 192 | miR-33a | moderate | moderate |
| 21 | miR-125a-3p | low | low | 193 | miR-33a* | low | low |
| 22 | miR-125a-5p | relatively low | relatively low | 194 | miR-33b | moderate | moderate |
| 23 | miR-125b-1* | low | low | 195 | miR-33b* | low | low |
| 24 | miR-125b-2* | moderate | moderate | 196 | miR-340* | low | low |
| 25 | miR-126* | relatively low | moderate | 197 | miR-342-3p | moderate | moderate |
| 26 | miR-1260 | relatively low | moderate | 198 | miR-342-5p | relatively low | moderate |
| 27 | miR-1260b | relatively low | moderate | 199 | miR-34a | moderate | relatively high |
| 28 | miR-1262 | low | low | 200 | miR-34a* | low | relatively low |
| 29 | miR-1266 | low | low | 201 | miR-34b* | low | relatively low |
| 30 | miR-1271 | low | low | 202 | miR-34c-5p | low | relatively low |
| 31 | miR-127-3p | low | low | 203 | miR-3605-5 p | low | low |
| 32 | miR-1274b | moderate | moderate | 204 | miR-3613-5 p | low | low |
| 33 | miR-1275 | low | relatively low | 205 | miR-361-3p | low | relatively low |
| 34 | miR-1280 | relatively high | relatively high | 206 | miR-3614-5 p | low | low |
| 35 | miR-1287 | relatively low | relatively low | 207 | miR-3615 | low | low |
| 36 | miR-1294 | low | low | 208 | miR-361-5p | moderate | moderate |
| 37 | miR-1296 | low | low | 209 | miR-362-3p | relatively low | relatively low |
| 38 | miR-1299 | low | relatively low | 210 | miR-362-5p | low | low |
| 39 | miR-1301 | low | low | 211 | miR-363 | relatively low | relatively low |
| 40 | miR-1303 | low | low | 212 | miR-365* | low | low |
| 41 | miR-130a | relatively low | moderate | 213 | miR-3656 | moderate | low |
| 42 | miR-130b | moderate | moderate | 214 | miR-3664 | low | low |
| 43 | miR-130b* | relatively low | relatively low | 215 | miR-3677 | low | low |
| 44 | miR-132 | moderate | moderate | 216 | miR-3688 | low | low |
| 45 | miR-132* | relatively low | moderate | 217 | miR-369-3p | low | low |
| 46 | miR-135a | relatively low | relatively low | 218 | miR-374a | moderate | moderate |
| 47 | miR-135b | low | low | 219 | miR-374a* | moderate | moderate |
| 48 | miR-136* | low | relatively low | 220 | miR-374b | moderate | moderate |
| 49 | miR-140-3p | moderate | relatively high | 221 | miR-374b* | low | low |
| 50 | miR-140-5p | relatively low | relatively low | 222 | miR-377 | low | low |
| 51 | miR-141* | relatively high | relatively high | 223 | miR-378* | relatively low | relatively low |
| 52 | miR-143* | low | relatively low | 224 | miR-381 | low | low |
| 53 | miR-144* | low | low | 225 | miR-382 | low | low |
| 54 | miR-145 | relatively low | relatively low | 226 | miR-383 | low | moderate |
| 55 | miR-1468 | low | low | 227 | miR-3909 | low | relatively low |
| 56 | miR-146b-3p | relatively high | relatively high | 228 | miR-3912 | relatively low | relatively low |
| 57 | miR-146b-5p | high | high | 229 | miR-3913 | low | relatively low |
| 58 | miR-147b | low | relatively low | 230 | miR-3919 | low | low |
| 59 | miR-148a | high | high | 231 | miR-3920 | low | low |
| 60 | miR-148a* | relatively high | high | 232 | miR-3942 | low | low |
| 61 | miR-148b | moderate | moderate | 233 | miR-409-3p | low | low |
| 62 | miR-148b* | moderate | moderate | 234 | miR-410 | low | relatively low |
| 63 | miR-151-3p | relatively high | relatively high | 235 | miR-421 | moderate | moderate |
| 64 | miR-151-5p | moderate | moderate | 236 | miR-422a | relatively low | relatively low |
| 65 | miR-1537 | relatively low | moderate | 237 | miR-423-3p | relatively high | relatively high |
| 66 | miR-155* | low | low | 238 | miR-423-5p | relatively low | moderate |
| 67 | miR-15a | moderate | moderate | 239 | miR-424 | low | relatively low |
| 68 | miR-15a* | low | low | 240 | miR-425* | moderate | moderate |
| 69 | miR-15b | moderate | moderate | 241 | miR-4284 | low | low |
| 70 | miR-15b* | low | low | 242 | miR-4286 | relatively low | relatively low |
| 71 | miR-16-1* | low | low | 243 | miR-4326 | low | low |
| 72 | miR-16-2* | relatively low | moderate | 244 | miR-449a | low | low |
| 73 | miR-17* | relatively low | moderate | 245 | miR-449b | low | low |
| 74 | miR-181a* | moderate | relatively high | 246 | miR-450a | low | relatively low |
| 75 | miR-181a-2* | relatively high | relatively high | 247 | miR-450b-5 p | low | relatively low |
| 76 | miR-181c | relatively high | relatively high | 248 | miR-454 | relatively low | relatively low |
| 77 | miR-181c* | moderate | moderate | 249 | miR-454* | relatively low | moderate |
| 78 | miR-181d | relatively low | moderate | 250 | miR-455-3p | low | low |
| 79 | miR-182* | low | low | 251 | miR-455-5p | moderate | moderate |
| 80 | miR-183* | low | low | 252 | miR-486-5p | relatively low | relatively low |
| 81 | miR-185* | low | relatively low | 253 | miR-489 | low | low |
| 82 | miR-187 | low | low | 254 | miR-493 | low | low |
| 83 | miR-188-3p | low | low | 255 | miR-499-3p | relatively low | low |
| 84 | miR-188-5p | low | low | 256 | miR-499-5p | moderate | moderate |
| 85 | miR-18a | moderate | relatively low | 257 | miR-500a | low | low |
| 86 | miR-18a* | relatively low | relatively low | 258 | miR-500a* | relatively low | relatively low |
| 87 | miR-190 | relatively low | relatively low | 259 | miR-500b | low | low |
| 88 | miR-190b | relatively low | relatively low | 260 | miR-501-3p | relatively low | moderate |
| 89 | miR-191 | high | high | 261 | miR-501-5p | low | low |
| 90 | miR-192* | low | low | 262 | miR-502-3p | low | relatively low |
| 91 | miR-193a-3p | relatively high | relatively high | 263 | miR-502-5p | low | low |
| 92 | miR-193a-5p | relatively low | relatively low | 264 | miR-504 | low | low |
| 93 | miR-193b | relatively high | relatively high | 265 | miR-505 | low | relatively low |
| 94 | miR-194 | relatively low | relatively low | 266 | miR-511 | moderate | relatively low |
| 95 | miR-195* | low | relatively low | 267 | miR-512-3p | relatively low | low |
| 96 | miR-196a | moderate | moderate | 268 | miR-515-5p | low | low |
| 97 | miR-196a* | low | low | 269 | miR-517a | relatively low | low |
| 98 | miR-196b | moderate | relatively low | 270 | miR-517b | relatively low | low |
| 99 | miR-197 | relatively low | moderate | 271 | miR-519a | relatively low | low |
| 100 | miR-199a-3p | relatively low | relatively low | 272 | miR-519c-3p | relatively low | low |
| 101 | miR-199b-3p | relatively low | relatively low | 273 | miR-520a-3 p | low | low |
| 102 | miR-199b-5p | low | relatively low | 274 | miR-532-3p | low | low |
| 103 | miR-19a | relatively high | high | 275 | miR-532-5p | moderate | moderate |
| 104 | miR-19b | relatively high | high | 276 | miR-542-3p | relatively low | relatively low |
| 105 | miR-200a | relatively high | relatively high | 277 | miR-548c-5p | low | relatively low |
| 106 | miR-200a* | relatively high | moderate | 278 | miR-548d-5p | low | relatively low |
| 107 | miR-200b | relatively high | relatively high | 279 | miR-548e | low | low |
| 108 | miR-200b* | moderate | relatively low | 280 | miR-548h | low | low |
| 109 | miR-200c | relatively high | moderate | 281 | miR-548i | relatively low | moderate |
| 110 | miR-200c* | low | low | 282 | miR-548j | low | low |
| 111 | miR-20a | relatively high | relatively high | 283 | miR-548k | relatively low | moderate |
| 112 | miR-20a* | relatively low | relatively low | 284 | miR-548n | low | low |
| 113 | miR-20b | low | low | 285 | miR-548o | low | relatively low |
| 114 | miR-21* | relatively high | high | 286 | miR-548p | relatively low | relatively low |
| 115 | miR-2114 | low | low | 287 | miR-548y | low | low |
| 116 | miR-2114* | low | low | 288 | miR-548z | low | low |
| 117 | miR-2115 | low | relatively low | 289 | miR-550a* | low | low |
| 118 | miR-2115* | low | relatively low | 290 | miR-551a | low | relatively low |
| 119 | miR-2116 | low | low | 291 | miR-574-3p | moderate | moderate |
| 120 | miR-215 | relatively low | relatively low | 292 | miR-574-5p | low | relatively low |
| 121 | miR-219-1-3 p | low | low | 293 | miR-576-3p | low | relatively low |
| 122 | miR-219-5p | low | low | 294 | miR-576-5p | relatively low | relatively low |
| 123 | miR-22* | relatively low | relatively low | 295 | miR-577 | moderate | moderate |
| 124 | miR-221* | relatively low | relatively low | 296 | miR-579 | low | low |
| 125 | miR-223* | relatively low | relatively low | 297 | miR-580 | low | low |
| 126 | miR-224 | moderate | relatively low | 298 | miR-582-3p | moderate | moderate |
| 127 | miR-224* | low | low | 299 | miR-582-5p | relatively low | relatively low |
| 128 | miR-2277-5p | low | low | 300 | miR-589 | low | low |
| 129 | miR-2355-3p | low | relatively low | 301 | miR-590-3p | moderate | moderate |
| 130 | miR-2355-5p | low | relatively low | 302 | miR-590-5p | relatively low | relatively low |
| 131 | miR-23a | moderate | relatively high | 303 | miR-597 | low | low |
| 132 | miR-23b | moderate | relatively high | 304 | miR-598 | moderate | moderate |
| 133 | miR-24-2* | relatively low | relatively low | 305 | miR-616* | low | relatively low |
| 134 | miR-26a | high | high | 306 | miR-618 | low | relatively low |
| 135 | miR-26a-1* | low | low | 307 | miR-624* | low | low |
| 136 | miR-26b | relatively high | relatively high | 308 | miR-625 | low | low |
| 137 | miR-26b* | relatively low | moderate | 309 | miR-625* | low | relatively low |
| 138 | miR-27a | moderate | relatively high | 310 | miR-627 | low | relatively low |
| 139 | miR-27a* | relatively low | moderate | 311 | miR-628-3p | low | low |
| 140 | miR-27b | relatively high | high | 312 | miR-628-5p | relatively low | relatively low |
| 141 | miR-27b* | low | relatively low | 313 | miR-629 | relatively low | moderate |
| 142 | miR-28-3p | relatively high | relatively high | 314 | miR-629* | low | relatively low |
| 143 | miR-28-5p | moderate | moderate | 315 | miR-642a | low | low |
| 144 | miR-296-5p | low | relatively low | 316 | miR-651 | relatively low | relatively low |
| 145 | miR-29a | relatively high | relatively high | 317 | miR-660 | relatively high | relatively high |
| 146 | miR-29a* | low | relatively low | 318 | miR-671-3p | relatively low | low |
| 147 | miR-29b | relatively low | relatively low | 319 | miR-671-5p | low | low |
| 148 | miR-29b-2* | low | low | 320 | miR-7 | low | low |
| 149 | miR-29c | relatively high | relatively high | 321 | miR-708* | low | low |
| 150 | miR-29c* | low | low | 322 | miR-7-1* | relatively low | relatively low |
| 151 | miR-301a | moderate | moderate | 323 | miR-720 | low | relatively low |
| 15 2 | miR-301b | relatively low | relatively low | 324 | miR-744 | low | low |
| 153 | miR-30a | high | high | 325 | miR-744* | low | low |
| 154 | miR-30a* | relatively high | relatively high | 326 | miR-766 | low | relatively low |
| 155 | miR-30b | high | relatively high | 327 | miR-769-3p | low | low |
| 156 | miR-30b* | low | low | 328 | miR-769-5p | moderate | moderate |
| 157 | miR-30c | relatively high | relatively high | 329 | miR-873 | low | relatively low |
| 158 | miR-30c-1* | low | low | 330 | miR-885-5p | relatively low | relatively low |
| 159 | miR-30c-2* | relatively low | relatively low | 331 | miR-889 | low | low |
| 160 | miR-30d | high | high | 332 | miR-891a | low | low |
| 16 1 | miR-30d* | relatively low | moderate | 333 | miR-9 | low | low |
| 162 | miR-30e | relatively high | high | 334 | miR-92a-1* | relatively low | relatively low |
| 163 | miR-30e* | relatively high | relatively high | 335 | miR-92b | moderate | moderate |
| 164 | miR-3116 | low | low | 336 | miR-93* | low | relatively low |
| 165 | miR-3127 | low | low | 337 | miR-934 | low | relatively low |
| 166 | miR-3130-3p | low | low | 338 | miR-937 | relatively low | low |
| 167 | miR-3130-5p | low | low | 339 | miR-941 | moderate | moderate |
| 168 | miR-3133 | low | low | 340 | miR-944 | low | relatively low |
| 169 | miR-3144-3p | low | low | 341 | miR-99a | relatively high | high |
| 170 | miR-3158 | low | relatively low | 342 | miR-99a* | moderate | relatively low |
| 171 | miR-3182 | low | relatively low | 343 | miR-99b | relatively high | relatively high |
| 172 | miR-3194 | low | low | 344 | miR-99b* | relatively low | relatively low |

Figure 1 is the analysis for the differential expression of microRNAs in colostrum and mature breast milk, wherein R²=0.8897 (far less than 0.99), which demonstrates that there is difference in the expression profiles of the microRNAs in colostrum and mature breast milk. Therefore, an unified standard for detecting human milk microRNAs can be established in combination with the detection results of the microRNAs in human mature breast milk and colostrum.

### Example 3: microRNAs having difference in expression in human mature breast milk and colostrum

**Comprehensive** expression profiles of the microRNAs in human milk are established in this example in combination with the Solexa sequencing results of the microRNAs having difference in expression in human mature breast milk and colostrum.

The specific steps for Solexa sequencing are as described in example 1 with the exception that the collected samples are human mature breast milk and colostrum.

The particular results are as follows:

Table 3 comprises the microRNAs having difference in expression in human mature breast milk and colostrum. It can be seen from the results that 115 kinds of microRNAs have differential expression, wherein the contents of 94 kinds of microRNAs are relatively high in human colostrum, but the contents of the other 21 kinds of microRNAs are relatively low in human colostrum. The results showed that the contents of the majority of microRNAs in human milk decrease along with the lactation process.

**Table 3 microRNAs having difference in expression in human mature breast milk and colostrum**

| No. | miR variety | miR content (human mature breast milk) | miR content (human colostrum) | No. | miR variety | miR content (human mature breast milk) | miR content (human colostrum) |
|---|---|---|---|---|---|---|---|
| 1 | let-7a* | relatively low | moderate | 58 | miR-320 0-3p | relatively low | low |
| 2 | let-7b* | relatively low | moderate | 59 | miR-320 a | moderate | relatively high |
| 3 | let-7d* | relatively low | moderate | 60 | miR-320 b | low | relatively low |
| 4 | let-7f-1* | relatively low | moderate | 61 | miR-320 c | low | relatively low |
| 5 | let-7f-2* | relatively low | moderate | 62 | miR-320 d | low | relatively low |
| 6 | let-7g | high | relatively high | 63 | miR-331- 3p | low | relatively low |
| 7 | let-7g* | relatively low | moderate | 64 | miR-338- 3p | low | relatively low |
| 8 | miR-10b | low | relatively low | 65 | miR-339- 5p | relatively low | low |
| 9 | miR-124 6 | relatively low | moderate | 66 | miR-342- 5p | relatively low | moderate |
| 10 | miR-126* | relatively low | moderate | 67 | miR-34a | moderate | relatively high |
| 11 | miR-126 0 | relatively low | moderate | 68 | miR-34a* | low | relatively low |
| 12 | miR-126 0b | relatively low | moderate | 69 | miR-34b* | low | relatively low |
| 13 | miR-127 5 | low | relatively low | 70 | miR-34c- 5p | low | relatively low |
| 14 | miR-129 9 | low | relatively low | 71 | miR-361- 3p | low | relatively low |
| 15 | miR-130 a | relatively low | moderate | 72 | miR-365 6 | moderate | low |
| 16 | miR-132* | relatively low | moderate | 73 | miR-383 | low | moderate |
| 17 | miR-136* | low | relatively low | 74 | miR-390 9 | low | relatively low |
| 18 | miR-140-3p | moderate | relatively high | 75 | miR-3913 | low | relatively low |
| 19 | miR-143* | low | relatively low | 76 | miR-410 | low | relatively low |
| 20 | miR-147 b | low | relatively low | 77 | miR-423-5p | relatively low | moderate |
| 21 | miR-148 a* | relatively high | high | 78 | miR-424 | low | relatively low |
| 22 | miR-153 7 | relatively low | moderate | 79 | miR-450 a | low | relatively low |
| 23 | miR-16-2 * | relatively low | moderate | 80 | miR-450 b-5p | low | relatively low |
| 24 | miR-17* | relatively low | moderate | 81 | miR-454* | relatively low | moderate |
| 25 | miR-181 a* | moderate | relatively high | 82 | miR-499-3p | relatively low | low |
| 26 | miR-181 d | relatively low | moderate | 83 | miR-501-3p | relatively low | moderate |
| 27 | miR-185* | low | relatively low | 84 | miR-502-3p | low | relatively low |
| 28 | miR-18a | moderate | relatively low | 85 | miR-505 | low | relatively low |
| 29 | miR-195* | low | relatively low | 86 | miR-511 | moderate | relatively low |
| 30 | miR-196 b | moderate | relatively low | 87 | miR-512-3p | relatively low | low |
| 31 | miR-197 | relatively low | moderate | 88 | miR-517 a | relatively low | low |
| 32 | miR-199 b-5p | low | relatively low | 89 | miR-517 b | relatively low | low |
| 33 | miR-19a | relatively high | high | 90 | miR-519 a | relatively low | low |
| 34 | miR-19b | relatively high | high | 91 | miR-519 c-3p | relatively low | low |
| 35 | miR-200 a* | relatively high | moderate | 92 | miR-548 c-5p | low | relatively low |
| 36 | miR-200 b* | moderate | relatively low | 93 | miR-548 d-5p | low | relatively low |
| 37 | miR-200 c | relatively high | moderate | 94 | miR-548i | relatively low | moderate |
| 38 | miR-21* | relatively high | high | 95 | miR-548k | relatively low | moderate |
| 39 | miR-2115 | low | relatively low | 96 | miR-548o | low | relatively low |
| 40 | miR-2115* | low | relatively low | 97 | miR-551 a | low | relatively low |
| 41 | miR-224 | moderate | relatively low | 98 | miR-574-5p | low | relatively low |
| 42 | miR-235 5-3p | low | relatively low | 99 | miR-576-3p | low | relatively low |
| 43 | miR-235 5-5p | low | relatively low | 100 | miR-616* | low | relatively low |
| 44 | miR-23a | moderate | relatively high | 101 | miR-618 | low | relatively low |
| 45 | miR-23b | moderate | relatively high | 102 | miR-625* | low | relatively low |
| 46 | miR-26b* | relatively low | moderate | 103 | miR-627 | low | relatively low |
| 47 | miR-27a | moderate | relatively high | 104 | miR-629 | relatively low | moderate |
| 48 | miR-27a* | relatively low | moderate | 105 | miR-629* | low | relatively low |
| 49 | miR-27b | relatively high | high | 106 | miR-671-3p | relatively low | low |
| 50 | miR-27b* | low | relatively low | 107 | miR-720 | low | relatively low |
| 51 | miR-296-5p | low | relatively low | 108 | miR-766 | low | relatively low |
| 52 | miR-29a* | low | relatively low | 109 | miR-873 | low | relatively low |
| 53 | miR-30b | high | relatively high | 110 | miR-93* | low | relatively low |
| 54 | miR-30d* | relatively low | moderate | 111 | miR-934 | low | relatively low |
| 55 | miR-30e | relatively high | high | 112 | miR-937 | relatively low | low |
| 56 | miR-3158 | low | relatively low | 113 | miR-944 | low | relatively low |
| 57 | miR-318 2 | low | relatively low | 114 | miR-99a | relatively high | high |
| | | | | 115 | miR-99a* | moderate | relatively low |

The majority of microRNAs existing in human mature breast milk and colostrum are related to immune system and nerve system. It can be seen from the results that almost all of the microRNAs having large copy numbers in human milk especially in colostrum are related to immunity or nerves, for instance, miR-181a, miR-223, miR-146a, miR-16, miR-21, miR-30a, miR-19a, miR-19b and the like. It is indicated that the microRNAs having large copy numbers in human milk, especially in colostrum, likely exert biological functions in the development of the immune system and nerve system of infants.

### Example 4: relations between human milk microRNAs and nerve system

This example demonstrates the effects of human milk microRNAs (such as miR-19a) in nerve system (such as nerve cells).

ESR1 is a transcription factor with expression thereof induced by a ligand and relates to the differentiation of nerve cells. The reintroduction of ESR1 gene results in significant cell cycle arrest in neuroblastoma cells, which is not conducive to the differentiation of nerve cells.

### 4.1 miR-19a negatively regulates estrogen receptor α (ESR1)

Luciferase detection method is employed to verify the negative regulation of miR-19a on estrogen receptor α (ESR1), the specific steps of the method being:
(1) constructing luciferase expression plasmids containing Pre-miR-19a and 3' non-coding fluorescence reporter gene structure of ESR1 (3'-ESR1-wt), and 3'-ESR1-wt, respectively;
(2) screening positive clones and sequencing;
(3) amplifying the clones and purifying the plasmids for use;
(4) culturing HEK-293 cells, inoculating same to 24-well plates, and allowing growth for 10-24 h;
(5) cotransfecting luciferase expression plasmids to HEK-293 cells;
(6) adding luciferase substrate, allowing luciferase to react with the substrate to produce fluorescein, and determining the activity of luciferase through detecting the intensity of fluorescence.

The particular results are shown in figure 2. Figure 2 is the luciferase activity of the HEK-293 cells transfected with Pre-miR-19a and 3'-ESR1-wt. In this figure, *** represents P < 0.1%.

It can be seen from figure 2 that the luciferase activity of the HEK-293 cells transfected with Pre-miR-19a and 3'-ESR1-wt decrease very significantly compared with the control group, and miR-19a reduces the luciferase activity of 3'-ESR1-wt by approximately 40%. The results indicate that miR-19a reduces the luciferase activity of ESR1 and inhibits the expression of ESR1. miR-19a negatively regulates the expression of ESR1.

### 4.2 Effects of ESR1 expression in nerve cells

ESR1 is a transcription factor with expression thereof induced by a ligand and relates to the differentiation of nerve cells. miR-19a negatively regulates the expression of ESR1, then the effects of ESR1 expression on nerve cells are studied.

Firstly, a lentivirus expression vector (Lenti-ESR1) is constructed, ESR1 cDNA is expressed using the lentivirus structure, and then human neuroblastoma cells--SK-N-BE(2) cells are transfected. The specific steps are as follows:
(1) constructing the lentivirus expression vector (Lenti-ESR1) according to ESR1 sequence;
(2) for recombinant plasmids with correct sequence, extracting and purifying the recombinant plasmids having high quality and free of endotoxin;
(3) co-transfecting SK-N-BE(2) cells with efficient recombinant lentivirus expression vector (Lenti-ESR1) and lentiviral packaging empty plasmid vector (Lenti-Empty Vector) for virus packaging and production, and collecting virus liquid;
(4) concentrating and purifying the virus liquid;
(5) allowing high-quality virus liquid to subject to subsequent Western blot experiment and cell proliferation assay.

The expression of ESR1 in SK-N-BE(2) cells transfected with Lenti-ESR1 and Lenti-Empty Vector is detected using Western blot method. The specific steps are as follows:
(1) collection of protein samples: lysing the cultured cell samples using a lysing solution, treating same with ultrasound, and centrifuging same at 4°C under 12000×g for 10 minutes. The supernatant is removed and the protein concentration is determined using BCA method;
(2) electrophoresis: separation using 10% SDS-polyacrylamide gel electrophoresis (SDS-PAGE);
(3) membrane transfer: using polyvinylidene difluoride (PVDF) membrane;
(4) blockage: blocking same at 4°C overnight using 5% Tris-buffered physiological saline (TBS) plus Tween-20 (TBST) . The membrane is washed with TBST for 3 times with each time of 10 minutes.
(5) incubation of antibody: firstly diluting same with TBST of appropriate proportion and washing the membrane thoroughly; then labeling secondary antibody using horseradish peroxidase (HRP) diluted in appropriate proportion;
(6) detection of protein: detecting the protein using an enhanced chemiluminescence reagent;
   scanning same with Bandscan software and quantifying the autoradiographic intensity of each waveband.

Cell proliferation is analysed and detected using a flow cytometer after transfecting the cells with Lenti-ESR1 and Lenti-Empty Vector. The specific steps are as follows:
(1) collecting the culture fluids of the cells transfected with Lenti-ESR1 and Lenti-Empty Vector in a flow cytometry tube;
(2) adding 1 ml PBS buffer to wash once and adding the washing liquid to the tube;
(3) washing the remaining cells with 1 ml PBS buffer once again and adding the washing liquid to the centrifuge tube;
(4) centrifuging and discarding the supernatant; adding 1 ml PBS buffer to resuspend the cells, centrifuging again and discarding the resulting supernatant;
(5) finally, resuspending the cells in 500 microliters of PBS buffer;
(6) adding a nucleic acid dye to the cell suspension, gently and evenly mixing, incubating same on ice away from light for 10 minutes, and detecting cell proliferation using a flow cytometer.

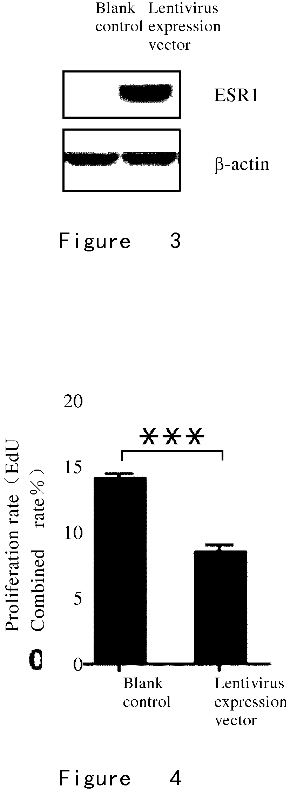

The particular results were shown in figure 3 and figure 4.

Figure 3 is the Western blot result for the expression of ESR1 in GFP positive SK-N-BE (2) cells transfected with Lenti-ESR1 and Lenti-Empty Vector. β-actin is used as the internal reference, and ESR1 almost has no expression in GFP positive SK-N-BE (2) cells transfected with Lenti-Empty Vector. The cells transfected with Lenti-Empty Vector are used as blank control, and the expression of ESR1 in GFP positive SK-N-BE(2) cells transfected with Lenti-ESR1 significantly increases.

Figure 4 is the flow cytometry analysis result for the cell proliferation after transfecting the cells with Lenti-ESR1 and Lenti-Empty Vector. In this figure, *** represents P < 0.1%. The cells transfected with Lenti-Empty Vector are used as blank control, and the expression of ESR1 in SK-N-BE(2) cells transfected with Lenti-ESR1 leads to the extremely significant decrease of cell proliferation.

In summary, miR-19a inhibits the expression of ESR1, and the inhibition of ESR1 is conducive to the differentiation of nerve cells. It can be seen therefrom that miR-19a is conducive to the differentiation of nerve cells. The results indicated that human milk microRNAs are related with nerve system.

### Example 5: expression profiles of microRNAs in human milk

In this example, the varieties and contents of microRNAs in cow milk are detected, and the microRNAs are screened and compareed with the detection results of the human breast milk secreted in different periods (such as colostrum and mature breast milk) in example 2, thus forming the expression profiles of the microRNAs specific to human milk and differential expression profiles of the microRNAs in human milk in different periods.

The specific steps for Solexa sequencing are as described in example 1 with the exception that the collected samples are human mature breast milk, colostrum and cow milk, respectively.

The following different expression profiles of microRNAs are formed according to the detection results.

### 5.1 microRNA expression profiles specific to human milk

Said microRNA expression profiles specific to human milk refers to the microRNAs existing in human mature breast milk and/or human colostrum but not existing in cow milk, as shown in Table 4.

**Table 4**

| No. | miR variety | miR content (human mature breast milk) | miR content (human colostrum) | No. | miR variety | miR content (human mature breast milk) | miR content (human colostrum) |
|---|---|---|---|---|---|---|---|
| 1 | let-7a* | relatively low | moderate | 121 | miR-3664 | low | low |
| 2 | let-7b* | relatively low | moderate | 122 | miR-3677 | low | low |
| 3 | let-7d* | relatively low | moderate | 123 | miR-3688 | low | low |
| 4 | let-7e* | low | low | 124 | miR-374a* | moderate | moderate |
| 5 | let-7f-1* | relatively low | moderate | 125 | miR-374b* | low | low |
| 6 | let-7f-2* | relatively low | moderate | 126 | miR-377 | low | low |
| 7 | let-7g* | relatively low | moderate | 127 | miR-378* | relatively low | relatively low |
| 8 | let-7i* | relatively low | relatively low | 128 | miR-3909 | low | relatively low |
| 9 | miR-100* | low | low | 129 | miR-3912 | relatively low | relatively low |
| 10 | miR-101* | low | low | 130 | miR-3913 | low | relatively low |
| 11 | miR-103-2* | low | low | 131 | miR-3919 | low | low |
| 12 | miR-106b* | relatively high | relatively high | 132 | miR-3920 | low | low |
| 13 | miR-10a* | low | low | 133 | miR-3942 | low | low |
| 14 | miR-1251 | relatively low | relatively low | 134 | miR-409-3p | low | low |
| 15 | miR-125b-1* | low | low | 135 | miR-410 | low | relatively low |
| 16 | miR-125b-2* | moderate | moderate | 136 | miR-422a | relatively low | relatively low |
| 17 | miR-126* | relatively low | moderate | 137 | miR-425* | moderate | moderate |
| 18 | miR-1260b | relatively low | moderate | 138 | miR-4284 | low | low |
| 19 | miR-1262 | low | low | 139 | miR-4286 | relatively low | relatively low |
| 20 | miR-1266 | low | low | 140 | miR-4326 | low | low |
| 21 | miR-1271 | low | low | 141 | miR-449b | low | low |
| 22 | miR-1275 | low | relatively low | 142 | miR-454 | relatively low | relatively low |
| 23 | miR-1280 | relatively high | relatively high | 143 | miR-454* | relatively low | moderate |
| 24 | miR-1287 | relatively low | relatively low | 144 | miR-489 | low | low |
| 25 | miR-1294 | low | low | 145 | miR-493 | low | low |
| 26 | miR-1299 | low | relatively low | 146 | miR-499-3p | relatively low | low |
| 27 | miR-1301 | low | low | 147 | miR-500a | low | low |
| 28 | miR-1303 | low | low | 148 | miR-500a* | relatively low | relatively low |
| 29 | miR-130b* | relatively low | relatively low | 149 | miR-500b | low | low |
| 30 | miR-132* | relatively low | moderate | 150 | miR-501-5p | low | low |
| 31 | miR-135b | low | low | 151 | miR-502-5p | low | low |
| 32 | miR-136* | low | relatively low | 152 | miR-504 | low | low |
| 33 | miR-141* | relatively high | relatively high | 153 | miR-511 | moderate | relatively low |
| 34 | miR-143* | low | relatively low | 154 | miR-512-3p | relatively low | low |
| 35 | miR-144* | low | low | 155 | miR-515-5p | low | low |
| 36 | miR-1468 | low | low | 156 | miR-517a | relatively low | low |
| 37 | miR-146b-3p | relatively high | relatively high | 157 | miR-517b | relatively low | low |
| 38 | miR-148a* | relatively high | high | 158 | miR-519a | relatively low | low |
| 39 | miR-148b* | moderate | moderate | 159 | miR-519c-3p | relatively low | low |
| 40 | miR-1537 | relatively low | moderate | 160 | miR-520a-3p | low | low |
| 41 | miR-155* | low | low | 161 | miR-548c-5p | low | relatively low |
| 42 | miR-15a* | low | low | 162 | miR-548d-5p | low | relatively low |
| 43 | miR-15b* | low | low | 163 | miR-548e | low | low |
| 44 | miR-16-1* | low | low | 164 | miR-548h | low | low |
| 45 | miR-16-2* | relatively low | moderate | 165 | miR-548i | relatively low | moderate |
| 46 | miR-17* | relatively low | moderate | 166 | miR-548j | low | low |
| 47 | miR-181a* | moderate | relatively high | 167 | miR-548k | relatively low | moderate |
| 48 | miR-181a-2* | relatively high | relatively high | 168 | miR-548n | low | low |
| 49 | miR-181c* | moderate | moderate | 169 | miR-548o | low | relatively low |
| 50 | miR-182* | low | low | 170 | miR-548p | relatively low | relatively low |
| 51 | miR-183* | low | low | 171 | miR-548y | low | low |
| 52 | miR-185* | low | relatively low | 172 | miR-548z | low | low |
| 53 | miR-18a* | relatively low | relatively low | 173 | miR-550a* | low | low |
| 54 | miR-192* | low | low | 174 | miR-551a | low | relatively low |
| 55 | miR-193b | relatively high | relatively high | 175 | miR-576-3p | low | relatively low |
| 56 | miR-195* | low | relatively low | 176 | miR-576-5p | relatively low | relatively low |
| 57 | miR-196a* | low | low | 177 | miR-577 | moderate | moderate |
| 58 | miR-199b-5p | low | relatively low | 178 | miR-579 | low | low |
| 59 | miR-200a* | relatively high | moderate | 179 | miR-580 | low | low |
| 60 | miR-200b* | moderate | relatively low | 180 | miR-582-3p | moderate | moderate |
| 61 | miR-200c* | low | low | 181 | miR-589 | low | low |
| 62 | miR-20a* | relatively low | relatively low | 182 | miR-590-3p | moderate | moderate |
| 63 | miR-20b | low | low | 183 | miR-590-5p | relatively low | relatively low |
| 64 | miR-21* | relatively high | high | 184 | miR-597 | low | low |
| 65 | miR-2114 | low | low | 185 | miR-598 | moderate | moderate |
| 66 | miR-2114* | low | low | 186 | miR-616* | low | relatively low |
| 67 | miR-2115 | low | relatively low | 187 | miR-618 | low | relatively low |
| 68 | miR-2115* | low | relatively low | 188 | miR-624* | low | low |
| 69 | miR-2116 | low | low | 189 | miR-625 | low | low |
| 70 | miR-22* | relatively low | relatively low | 190 | miR-625* | low | relatively low |
| 71 | miR-221* | relatively low | relatively low | 191 | miR-627 | low | relatively low |
| 72 | miR-223* | relatively low | relatively low | 192 | miR-629 | relatively | moderate |
| | | | | | | low | |
| 73 | miR-224* | low | low | 193 | miR-629* | low | relatively low |
| 74 | miR-2277-5p | low | low | 194 | miR-642a | low | low |
| 75 | miR-2355-3p | low | relatively low | 195 | miR-651 | relatively low | relatively low |
| 76 | miR-2355-5p | low | relatively low | 196 | miR-708* | low | low |
| 77 | miR-24-2* | relatively low | relatively low | 197 | miR-7-1* | relatively low | relatively low |
| 78 | miR-26a-1* | low | low | 198 | miR-744* | low | low |
| 79 | miR-26b* | relatively low | moderate | 199 | miR-766 | low | relatively low |
| 80 | miR-27a* | relatively low | moderate | 200 | miR-769-3p | low | low |
| 81 | miR-27b* | low | relatively low | 201 | miR-769-5p | moderate | moderate |
| 82 | miR-28-3p | relatively high | relatively high | 202 | miR-873 | low | relatively low |
| 83 | miR-296-5p | low | relatively low | 203 | miR-889 | low | low |
| 84 | miR-29a* | low | relatively low | 204 | miR-891a | low | low |
| 85 | miR-29b-2* | low | low | 205 | miR-92a-1* | relatively low | relatively low |
| 86 | miR-29c* | low | low | 206 | miR-93* | low | relatively low |
| 87 | miR-301b | relatively low | relatively low | 207 | miR-934 | low | relatively low |
| 88 | miR-30a* | relatively high | relatively high | 208 | miR-937 | relatively low | low |
| 89 | miR-30b* | low | low | 209 | miR-941 | moderate | moderate |
| 90 | miR-30c-1* | low | low | 210 | miR-944 | low | relatively low |
| 91 | miR-30c-2* | relatively low | relatively low | 211 | miR-99a* | moderate | relatively low |
| 92 | miR-30d* | relatively low | moderate | 212 | miR-99b* | relatively low | relatively low |
| 93 | miR-30e* | relatively high | relatively high | 213 | miR-10b | low | relatively low |
| 94 | miR-3116 | low | low | 214 | miR-1260 | relatively low | moderate |
| 95 | miR-3127 | low | low | 215 | miR-140-5p | relatively low | relatively low |
| 96 | miR-3130-3p | low | low | 216 | miR-147b | low | relatively low |
| 97 | miR-3130-5p | low | low | 217 | miR-181c | relatively high | relatively high |
| 98 | miR-3133 | low | low | 218 | miR-187 | low | low |
| 99 | miR-3144-3p | low | low | 219 | miR-188-3p | low | low |
| 100 | miR-3158 | low | relatively low | 220 | miR-188-5p | low | low |
| 101 | miR-3182 | low | relatively low | 221 | miR-18a | moderate | relatively low |
| 102 | miR-3194 | low | low | 222 | miR-190 | relatively low | relatively low |
| 103 | miR-3196 | low | low | 223 | miR-190b | relatively low | relatively low |
| 104 | miR-32 | relatively low | relatively low | 224 | miR-19a | relatively high | high |
| 105 | miR-32* | low | low | 225 | miR-219-1-3p | low | low |
| 106 | miR-3200-3p | relatively low | low | 226 | miR-219-5p | low | low |
| 107 | miR-335* | relatively high | relatively high | 227 | miR-301a | moderate | moderate |
| 108 | miR-339-3p | low | low | 228 | miR-330-5p | low | low |
| 109 | miR-33a* | low | low | 229 | miR-338-3p | low | relatively low |
| 110 | miR-33b* | low | low | 230 | miR-34c-5p | low | relatively low |
| 111 | miR-340* | low | low | 231 | miR-381 | low | low |
| 112 | miR-342-5p | relatively low | moderate | 232 | miR-383 | low | moderate |
| 113 | miR-34a* | low | relatively low | 233 | miR-424 | low | relatively low |
| 114 | miR-34b* | low | relatively low | 234 | miR-449a | low | low |
| 115 | miR-3605-5p | low | low | 235 | miR-450a | low | relatively low |
| 116 | miR-3613-5p | low | low | 236 | miR-455-5p | moderate | moderate |
| 117 | miR-3614-5p | low | low | 237 | miR-501-3p | relatively low | moderate |
| 118 | miR-3615 | low | low | 238 | miR-505 | low | relatively low |
| 119 | miR-365* | low | low | 239 | miR-542-3p | relatively low | relatively low |
| 120 | miR-3656 | moderate | low | 240 | miR-582-5p | relatively low | relatively low |
| | | | | 241 | miR-9 | low | low |

Infant food supplements can be prepared through adding specific contents of these microRNAs to cow milk products.

### 5.2 microRNA expression profiles specific to human colostrum

Said microRNA expression profiles specific to human colostrum refers to (1) the microRNAs having a higher content in human colostrum than in human mature breast milk, or (2) the microRNAs which are detectable in human colostrum but not detectable or do not exist in human mature breast milk, as shown in Table 5.

**Table 5**

| No. | miR variety | miR content (human mature breast milk) | miR content (human colostrum) | No. | miR variety | miR content (human mature breast milk) | miR content (human colostrum) |
|---|---|---|---|---|---|---|---|
| 1 | miR-148a* | relatively high | high | 49 | miR-185* | low | relatively low |
| 2 | miR-19a | relatively high | high | 50 | miR-195* | low | relatively low |
| 3 | miR-19b | relatively high | high | 51 | miR-199b-5p | low | relatively low |
| 4 | miR-21* | relatively high | high | 52 | miR-2115 | low | relatively low |
| 5 | miR-27b | relatively high | high | 53 | miR-2115* | low | relatively low |
| 6 | miR-30e | relatively high | high | 54 | miR-2355-3p | low | relatively low |
| 7 | miR-99a | relatively high | high | 55 | miR-2355-5p | low | relatively low |
| 8 | miR-140-3p | moderate | relatively high | 56 | miR-27b* | low | relatively low |
| 9 | miR-181a* | moderate | relatively high | 57 | miR-296-5p | low | relatively low |
| 10 | miR-23a | moderate | relatively high | 58 | miR-29a* | low | relatively low |
| 11 | miR-23b | moderate | relatively high | 59 | miR-3158 | low | relatively low |
| 12 | miR-27a | moderate | relatively high | 60 | miR-3182 | low | relatively low |
| 13 | miR-320a | moderate | relatively high | 61 | miR-320b | low | relatively low |
| 14 | miR-34a | moderate | relatively high | 62 | miR-320c | low | relatively low |
| 15 | let-7a* | relatively low | moderate | 63 | miR-320d | low | relatively low |
| 16 | let-7b* | relatively low | moderate | 64 | miR-331-3p | low | relatively low |
| 17 | let-7d* | relatively low | moderate | 65 | miR-338-3p | low | relatively low |
| 18 | let-7f-1* | relatively low | moderate | 66 | miR-34a* | low | relatively low |
| 19 | let-7f-2* | relatively low | moderate | 67 | miR-34b* | low | relatively low |
| 20 | let-7g* | relatively low | moderate | 68 | miR-34c-5p | low | relatively low |
| 21 | miR-1246 | relatively low | moderate | 69 | miR-361-3p | low | relatively low |
| 22 | miR-126* | relatively low | moderate | 70 | miR-3909 | low | relatively low |
| 23 | miR-1260 | relatively low | moderate | 71 | miR-3913 | low | relatively low |
| 24 | miR-1260b | relatively low | moderate | 72 | miR-410 | low | relatively low |
| 25 | miR-130a | relatively low | moderate | 73 | miR-424 | low | relatively low |
| 26 | miR-132* | relatively low | moderate | 74 | miR-450a | low | relatively low |
| 27 | miR-1537 | relatively low | moderate | 75 | miR-450b-5p | low | relatively low |
| 28 | miR-16-2* | relatively low | moderate | 76 | miR-502-3p | low | relatively low |
| 29 | miR-17* | relatively low | moderate | 77 | miR-505 | low | relatively low |
| 30 | miR-181d | relatively low | moderate | 78 | miR-548c-5p | low | relatively low |
| 31 | miR-197 | relatively low | moderate | 79 | miR-548d-5p | low | relatively low |
| 32 | miR-26b* | relatively low | moderate | 80 | miR-548o | low | relatively low |
| 33 | miR-27a* | relatively low | moderate | 81 | miR-551a | low | relatively low |
| 34 | miR-30d* | relatively low | moderate | 82 | miR-574-5p | low | relatively low |
| 35 | miR-342-5p | relatively low | moderate | 83 | miR-576-3p | low | relatively low |
| 36 | miR-423-5p | relatively low | moderate | 84 | miR-616* | low | relatively low |
| 37 | miR-454* | relatively low | moderate | 85 | miR-618 | low | relatively low |
| 38 | miR-501-3p | relatively low | moderate | 86 | miR-625* | low | relatively low |
| 39 | miR-548i | relatively low | moderate | 87 | miR-627 | low | relatively low |
| 40 | miR-548k | relatively low | moderate | 88 | miR-629* | low | relatively low |
| 41 | miR-629 | relatively low | moderate | 89 | miR-720 | low | relatively low |
| 42 | miR-383 | low | moderate | 90 | miR-766 | low | relatively low |
| 43 | miR-10b | low | relatively low | 91 | miR-873 | low | relatively low |
| 44 | miR-1275 | low | relatively low | 92 | miR-93* | low | relatively low |
| 45 | miR-1299 | low | relatively low | 93 | miR-934 | low | relatively low |
| 46 | miR-136* | low | relatively low | 94 | miR-944 | low | relatively low |
| 47 | miR-143* | low | relatively low | 95 | miR-145* | UD | relatively low |
| 48 | miR-147b | low | relatively low | 96 | miR-146a* | UD | relatively low |

### 5.3 microRNA expression profiles specific to human mature breast milk

Said microRNA expression profiles specific to human mature breast milk refers to (1) the microRNAs having a higher content in human mature breast milk than in human colostrum, or (2) the microRNAs which are detectable in human mature breast milk but not detectable or do not exist in human colostrum, as shown in Table 6.

**Table 6**

| No. | miR variety | miR content (human mature breast milk) | miR content (human colostrum) | No. | miR variety | miR content (human mature breast milk) | miR content (human colostrum) |
|---|---|---|---|---|---|---|---|
| 1 | let-7g | high | relatively high | 31 | miR-376a | low | UD |
| 2 | miR-30b | high | relatively high | 32 | miR-411 | low | UD |
| 3 | miR-200a* | relatively high | moderate | 33 | miR-4297 | low | UD |
| 4 | miR-200c | relatively high | moderate | 34 | miR-488 | low | UD |
| 5 | miR-18a | moderate | relatively low | 35 | miR-491-5p | low | UD |
| 6 | miR-196b | moderate | relatively low | 36 | miR-514 | low | UD |
| 7 | miR-200b* | moderate | relatively low | 37 | miR-516b | low | UD |
| 8 | miR-224 | moderate | relatively low | 38 | miR-517c | low | UD |
| 9 | miR-511 | moderate | relatively low | 39 | miR-518e | low | UD |
| 10 | miR-99a* | moderate | relatively low | 40 | miR-519a* | low | UD |
| 11 | miR-3656 | moderate | low | 41 | miR-519b-3p | low | UD |
| 12 | miR-1228* | low | UD | 42 | miR-520a-5p | low | UD |
| 13 | miR-1249 | low | UD | 43 | miR-520f | low | UD |
| 14 | miR-1276 | low | UD | 44 | miR-522 | low | UD |
| 15 | miR-1283 | low | UD | 45 | miR-524-3p | low | UD |
| 16 | miR-153 | low | UD | 46 | miR-548v | low | UD |
| 17 | miR-19b-1* | low | UD | 47 | miR-552 | low | UD |
| 18 | miR-2110 | low | UD | 48 | miR-589* | low | UD |
| 19 | miR-2116* | low | UD | 49 | miR-641 | low | UD |
| 20 | miR-2277-3p | low | UD | 50 | miR-643 | low | UD |
| 21 | miR-3065-3p | low | UD | 51 | miR-95 | low | UD |
| 22 | miR-3074 | low | UD | 52 | miR-3200-3p | relatively low | low |
| 23 | miR-3115 | low | UD | 53 | miR-339-5p | relatively low | low |
| 24 | miR-3174 | low | UD | 54 | miR-499-3p | relatively low | low |
| 25 | miR-3190 | low | UD | 55 | miR-512-3p | relatively low | low |
| 26 | miR-3200-5p | low | UD | 56 | miR-517a | relatively low | low |
| 27 | miR-326 | low | UD | 57 | miR-517b | relatively low | low |
| 28 | miR-3617 | low | UD | 58 | miR-519a | relatively low | low |
| 29 | miR-3620 | low | UD | 59 | miR-519c-3p | relatively low | low |
| 30 | miR-3654 | low | UD | 60 | miR-671-3p | relatively low | low |
| | | | | 61 | miR-937 | relatively low | low |

Based on the characteristics of microRNA profiles of 5.2 and 5.3, the scientific staging of infant foods can be guided, and milk products having different formulas can be prepared which are used as infant foods of different stages. 1. The milk products supplemented with the microRNAs having high content in human colostrum as described in Table 5 can be used in foods for infants of 0-1 month. 2. The milk products supplemented with the microRNAs having high content in human mature breast milk as described in Table 6 are used for foods for infants of 1-24 months.

### 5.4 microRNA expression profiles specific to cow milk

miR-148, miR-138, miR-146-5p, miR-523-5p, miR-523-3p, miR-885-3p, miR-935, miR-1308, and miR-1306 exist in cow milk, but do not in human milk. However, the majority of these microRNAs are upregulated in the bodies of cancer patients and function as negative regulation on cancers.

Therefore, the knockout of these microRNAs can inhibit the development of oncogenes. These microRNAs can be removed from milk products to prepare special milk products which would not affect genes or novel nutraceuticals for cancer patients.

All documents referred to in the present invention are incorporated by reference as if each reference is cited alone as a reference in the present application. In addition, it should be understood that after reading the teachings of the present invention described above, a skilled person in the art can make various changes or modifications of the invention, and these equivalent forms also fall into the scope as defined by the appended claims of the present application.

## Claims

1. An artificial milk product, wherein said milk product contains one or more kinds of human milk-derived microRNAs which are exogenously added, said human milk includes human colostrum or human mature breast milk.

2. The artificial milk product of claim 1, wherein said microRNAs are selected from the group consisting of:
(a) microRNAs specific to human colostrum and listed in Table A;
**Table A**
| No. | miR variety | No. | miR variety | No. | miR variety | No. | miR variety |
|---|---|---|---|---|---|---|---|
| 1 | miR-148a* | 25 | miR-130a | 49 | miR-185* | 73 | miR-424 |
| 2 | miR-19a | 26 | miR-132* | 50 | miR-195* | 74 | miR-450a |
| 3 | miR-19b | 27 | miR-1537 | 51 | miR-199b-5p | 75 | miR-450b-5p |
| 4 | miR-21* | 28 | miR-16-2* | 52 | miR-2115 | 76 | miR-502-3p |
| 5 | miR-27b | 29 | miR-17* | 53 | miR-2115* | 77 | miR-505 |
| 6 | miR-30e | 30 | miR-181d | 54 | miR-2355-3p | 78 | miR-548c-5p |
| 7 | miR-99a | 31 | miR-197 | 55 | miR-2355-5p | 79 | miR-548d-5p |
| 8 | miR-140-3p | 32 | miR-26b* | 56 | miR-27b* | 80 | miR-548o |
| 9 | miR-181a* | 33 | miR-27a* | 57 | miR-296-5p | 81 | miR-551a |
| 10 | miR-23a | 34 | miR-30d* | 58 | miR-29a* | 82 | miR-574-5p |
| 11 | miR-23b | 35 | miR-342-5p | 59 | miR-3158 | 83 | miR-576-3p |
| 12 | miR-27a | 36 | miR-423-5p | 60 | miR-3182 | 84 | miR-616* |
| 13 | miR-320a | 37 | miR-454* | 61 | miR-320b | 85 | miR-618 |
| 14 | miR-34a | 38 | miR-501-3p | 62 | miR-320c | 86 | miR-625* |
| 15 | let-7a* | 39 | miR-548i | 63 | miR-320d | 87 | miR-627 |
| 16 | let-7b* | 40 | miR-548k | 64 | miR-331-3p | 88 | miR-629* |
| 17 | let-7d* | 41 | miR-629 | 65 | miR-338-3p | 89 | miR-720 |
| 18 | let-7f-1* | 42 | miR-383 | 66 | miR-34a* | 90 | miR-766 |
| 19 | let-7f-2* | 43 | miR-10b | 67 | miR-34b* | 91 | miR-873 |
| 20 | let-7g* | 44 | miR-1275 | 68 | miR-34c-5p | 92 | miR-93* |
| 21 | miR-1246 | 45 | miR-1299 | 69 | miR-361-3p | 93 | miR-934 |
| 22 | miR-126* | 46 | miR-136* | 70 | miR-3909 | 94 | miR-944 |
| 23 | miR-1260 | 47 | miR-143* | 71 | miR-3913 | 95 | miR-145* |
| 24 | miR-1260b | 48 | miR-147b | 72 | miR-410 | 96 | miR-146a* |
(b) microRNAs specific to human mature breast milk and listed in Table B;
**Table B**
| No. | miR variety | No. | miR variety | No. | miR variety | No. | miR variety |
|---|---|---|---|---|---|---|---|
| 1 | let-7g | 16 | miR-153 | 31 | miR-376a | 46 | miR-548v |
| 2 | miR-30b | 17 | miR-19b-1* | 32 | miR-411 | 47 | miR-552 |
| 3 | miR-200a* | 18 | miR-2110 | 33 | miR-4297 | 48 | miR-589* |
| 4 | miR-200c | 19 | miR-2116* | 34 | miR-488 | 49 | miR-641 |
| 5 | miR-18a | 20 | miR-2277-3p | 35 | miR-491-5p | 50 | miR-643 |
| 6 | miR-196b | 21 | miR-3065-3p | 36 | miR-514 | 51 | miR-95 |
| 7 | miR-200b* | 22 | miR-3074 | 37 | miR-516b | 52 | miR-3200-3p |
| 8 | miR-224 | 23 | miR-3115 | 38 | miR-517c | 53 | miR-339-5p |
| 9 | miR-511 | 24 | miR-3174 | 39 | miR-518e | 54 | miR-499-3p |
| 10 | miR-99a* | 25 | miR-3190 | 40 | miR-519a* | 55 | miR-512-3p |
| 11 | miR-3656 | 26 | miR-3200-5p | 41 | miR-519b-3p | 56 | miR-517a |
| 12 | miR-1228* | 27 | miR-326 | 42 | miR-520a-5p | 57 | miR-517b |
| 13 | miR-1249 | 28 | miR-3617 | 43 | miR-520f | 58 | miR-519a |
| 14 | miR-1276 | 29 | miR-3620 | 44 | miR-522 | 59 | miR-519c-3p |
| 15 | miR-1283 | 30 | miR-3654 | 45 | miR-524-3p | 60 | miR-671-3p |
| | | | | | | 61 | miR-937 |
(c) common microRNAs existing in both human colostrum and mature breast milk and listed in Table C; and
**Table C**
| No. | miR variety | No. | miR variety | No. | miR variety | No. | miR variety |
|---|---|---|---|---|---|---|---|
| 1 | let-7a* | 87 | miR-190 | 173 | miR-3196 | 259 | miR-500b |
| 2 | let-7b* | 88 | miR-190b | 174 | miR-32 | 260 | miR-501-3p |
| 3 | let-7d* | 89 | miR-191 | 175 | miR-32* | 261 | miR-501-5p |
| 4 | let-7e* | 90 | miR-192* | 176 | miR-3200-3p | 262 | miR-502-3p |
| 5 | let-7f-1* | 91 | miR-193a-3p | 177 | miR-320a | 263 | miR-502-5p |
| 6 | let-7f-2* | 92 | miR-193a-5p | 178 | miR-320b | 264 | miR-504 |
| 7 | let-7g | 93 | miR-193b | 179 | miR-320c | 265 | miR-505 |
| 8 | let-7g* | 94 | miR-194 | 180 | miR-320d | 266 | miR-511 |
| 9 | let-7i* | 95 | miR-195* | 181 | miR-324-3p | 267 | miR-512-3p |
| 10 | miR-100* | 96 | miR-196a | 182 | miR-324-5p | 268 | miR-515-5p |
| 11 | miR-101* | 97 | miR-196a* | 183 | miR-330-3p | 269 | miR-517a |
| 12 | miR-103-2* | 98 | miR-196b | 184 | miR-330-5p | 270 | miR-517b |
| 13 | miR-106a | 99 | miR-197 | 185 | miR-331-3p | 271 | miR-519a |
| 14 | miR-106b | 100 | miR-199a-3p | 186 | miR-331-5p | 272 | miR-519c-3p |
| 15 | miR-106b* | 101 | miR-199b-3p | 187 | miR-335 | 273 | miR-520a-3p |
| 16 | miR-10a | 102 | miR-199b-5p | 188 | miR-335* | 274 | miR-532-3p |
| 17 | miR-10a* | 103 | miR-19a | 189 | miR-338-3p | 275 | miR-532-5p |
| 18 | miR-10b | 104 | miR-19b | 190 | miR-339-3p | 276 | miR-542-3p |
| 19 | miR-1246 | 105 | miR-200a | 191 | miR-339-5p | 277 | miR-548c-5p |
| 20 | miR-1251 | 106 | miR-200a* | 192 | miR-33a | 278 | miR-548d-5p |
| 21 | miR-125a-3p | 107 | miR-200b | 193 | miR-33a* | 279 | miR-548e |
| 22 | miR-125a-5p | 108 | miR-200b* | 194 | miR-33b | 280 | miR-548h |
| 23 | miR-125b-1* | 109 | miR-200c | 195 | miR-33b* | 281 | miR-548i |
| 24 | miR-125b-2* | 110 | miR-200c* | 196 | miR-340* | 282 | miR-548j |
| 25 | miR-126* | 111 | miR-20a | 197 | miR-342-3p | 283 | miR-548k |
| 26 | miR-1260 | 112 | miR-20a* | 198 | miR-342-5p | 284 | miR-548n |
| 27 | miR-1260b | 113 | miR-20b | 199 | miR-34a | 285 | miR-548o |
| 28 | miR-1262 | 114 | miR-21* | 200 | miR-34a* | 286 | miR-548p |
| 29 | miR-1266 | 115 | miR-2114 | 201 | miR-34b* | 287 | miR-548y |
| 30 | miR-1271 | 116 | miR-2114* | 202 | miR-34c-5p | 288 | miR-548z |
| 31 | miR-127-3p | 117 | miR-2115 | 203 | miR-3605-5p | 289 | miR-550a* |
| 32 | miR-1274b | 118 | miR-2115* | 204 | miR-3613-5p | 290 | miR-551a |
| 33 | miR-1275 | 119 | miR-2116 | 205 | miR-361-3p | 291 | miR-574-3p |
| 34 | miR-1280 | 120 | miR-215 | 206 | miR-3614-5p | 292 | miR-574-5p |
| 35 | miR-1287 | 121 | miR-219-1-3p | 207 | miR-3615 | 293 | miR-576-3p |
| 36 | miR-1294 | 122 | miR-219-5p | 208 | miR-361-5p | 294 | miR-576-5p |
| 37 | miR-1296 | 123 | miR-22* | 209 | miR-362-3p | 295 | miR-577 |
| 38 | miR-1299 | 124 | miR-221* | 210 | miR-362-5p | 296 | miR-579 |
| 39 | miR-1301 | 125 | miR-223* | 211 | miR-363 | 297 | miR-580 |
| 40 | miR-1303 | 126 | miR-224 | 212 | miR-365* | 298 | miR-582-3p |
| 41 | miR-130a | 127 | miR-224* | 213 | miR-3656 | 299 | miR-582-5p |
| 42 | miR-130b | 128 | miR-2277-5p | 214 | miR-3664 | 300 | miR-589 |
| 43 | miR-130b* | 129 | miR-2355-3p | 215 | miR-3677 | 301 | miR-590-3p |
| 44 | miR-132 | 130 | miR-2355-5p | 216 | miR-3688 | 302 | miR-590-5p |
| 45 | miR-132* | 131 | miR-23a | 217 | miR-369-3p | 303 | miR-597 |
| 46 | miR-135a | 132 | miR-23b | 218 | miR-374a | 304 | miR-598 |
| 47 | miR-135b | 133 | miR-24-2* | 219 | miR-374a* | 305 | miR-616* |
| 48 | miR-136* | 134 | miR-26a | 220 | miR-374b | 306 | miR-618 |
| 49 | miR-140-3p | 135 | miR-26a-1* | 221 | miR-374b* | 307 | miR-624* |
| 50 | miR-140-5p | 136 | miR-26b | 222 | miR-377 | 308 | miR-625 |
| 51 | miR-141* | 137 | miR-26b* | 223 | miR-378* | 309 | miR-625* |
| 52 | miR-143* | 138 | miR-27a | 224 | miR-381 | 310 | miR-627 |
| 53 | miR-144* | 139 | miR-27a* | 225 | miR-382 | 311 | miR-628-3p |
| 54 | miR-145 | 140 | miR-27b | 226 | miR-383 | 312 | miR-628-5p |
| 55 | miR-1468 | 141 | miR-27b* | 227 | miR-3909 | 313 | miR-629 |
| 56 | miR-146b-3p | 142 | miR-28-3p | 228 | miR-3912 | 314 | miR-629* |
| 57 | miR-146b-5p | 143 | miR-28-5p | 229 | miR-3913 | 315 | miR-642a |
| 58 | miR-147b | 144 | miR-296-5p | 230 | miR-3919 | 316 | miR-651 |
| 59 | miR-148a | 145 | miR-29a | 231 | miR-3920 | 317 | miR-660 |
| 60 | miR-148a* | 146 | miR-29a* | 232 | miR-3942 | 318 | miR-671-3p |
| 61 | miR-148b | 147 | miR-29b | 233 | miR-409-3p | 319 | miR-671-5p |
| 62 | miR-148b* | 148 | miR-29b-2* | 234 | miR-410 | 320 | miR-7 |
| 63 | miR-151-3p | 149 | miR-29c | 235 | miR-421 | 321 | miR-708* |
| 64 | miR-151-5p | 150 | miR-29c* | 236 | miR-422a | 322 | miR-7-1* |
| 65 | miR-1537 | 151 | miR-301a | 237 | miR-423-3p | 323 | miR-720 |
| 66 | miR-155* | 152 | miR-301b | 238 | miR-423-5p | 324 | miR-744 |
| 67 | miR-15a | 153 | miR-30a | 239 | miR-424 | 325 | miR-744* |
| 68 | miR-15a* | 154 | miR-30a* | 240 | miR-425* | 326 | miR-766 |
| 69 | miR-15b | 155 | miR-30b | 241 | miR-4284 | 327 | miR-769-3p |
| 70 | miR-15b* | 156 | miR-30b* | 242 | miR-4286 | 328 | miR-769-5p |
| 71 | miR-16-1* | 157 | miR-30c | 243 | miR-4326 | 329 | miR-873 |
| 72 | miR-16-2* | 158 | miR-30c-1* | 244 | miR-449a | 330 | miR-885-5p |
| 73 | miR-17* | 159 | miR-30c-2* | 245 | miR-449b | 331 | miR-889 |
| 74 | miR-181a* | 160 | miR-30d | 246 | miR-450a | 332 | miR-891a |
| 75 | miR-181a-2* | 161 | miR-30d* | 247 | miR-450b-5p | 333 | miR-9 |
| 76 | miR-181c | 162 | miR-30e | 248 | miR-454 | 334 | miR-92a-1* |
| 77 | miR-181c* | 163 | miR-30e* | 249 | miR-454* | 335 | miR-92b |
| 78 | miR-181d | 164 | miR-3116 | 250 | miR-455-3p | 336 | miR-93* |
| 79 | miR-182* | 165 | miR-3127 | 251 | miR-455-5p | 337 | miR-934 |
| 80 | miR-183* | 166 | miR-3130-3p | 252 | miR-486-5p | 338 | miR-937 |
| 81 | miR-185* | 167 | miR-3130-5p | 253 | miR-489 | 339 | miR-941 |
| 82 | miR-187 | 168 | miR-3133 | 254 | miR-493 | 340 | miR-944 |
| 83 | miR-188-3p | 169 | miR-3144-3p | 255 | miR-499-3p | 341 | miR-99a |
| 84 | miR-188-5p | 170 | miR-3158 | 256 | miR-499-5p | 342 | miR-99a* |
| 85 | miR-18a | 171 | miR-3182 | 257 | miR-500a | 343 | miR-99b |
| 86 | miR-18a* | 172 | miR-3194 | 258 | miR-500a* | 344 | miR-99b* |
and (d) microRNAs not existing in cow milk but existing in human colostrum or human mature breast milk, and listed in Table D.
**Table D**
| No. | miR variety | No. | miR variety | No. | miR variety | No. | miR variety |
|---|---|---|---|---|---|---|---|
| 1 | let-7a* | 61 | miR-200c* | 121 | miR-3664 | 181 | miR-589 |
| 2 | let-7b* | 62 | miR-20a* | 122 | miR-3677 | 182 | miR-590-3p |
| 3 | let-7d* | 63 | miR-20b | 123 | miR-3688 | 183 | miR-590-5p |
| 4 | let-7e* | 64 | miR-21* | 124 | miR-374a* | 184 | miR-597 |
| 5 | let-7f-1* | 65 | miR-2114 | 125 | miR-374b* | 185 | miR-598 |
| 6 | let-7f-2* | 66 | miR-2114* | 126 | miR-377 | 186 | miR-616* |
| 7 | let-7g* | 67 | miR-2115 | 127 | miR-378* | 187 | miR-618 |
| 8 | let-7i* | 68 | miR-2115* | 128 | miR-3909 | 188 | miR-624* |
| 9 | miR-100* | 69 | miR-2116 | 129 | miR-3912 | 189 | miR-625 |
| 10 | miR-101* | 70 | miR-22* | 130 | miR-3913 | 190 | miR-625* |
| 11 | miR-103-2* | 71 | miR-221* | 131 | miR-3919 | 191 | miR-627 |
| 12 | miR-106b* | 72 | miR-223* | 132 | miR-3920 | 192 | miR-629 |
| 13 | miR-10a* | 73 | miR-224* | 133 | miR-3942 | 193 | miR-629* |
| 14 | miR-1251 | 74 | miR-2277-5p | 134 | miR-409-3p | 194 | miR-642a |
| 15 | miR-125b-1* | 75 | miR-2355-3p | 135 | miR-410 | 195 | miR-651 |
| 16 | miR-125b-2* | 76 | miR-2355-5p | 136 | miR-422a | 196 | miR-708* |
| 17 | miR-126* | 77 | miR-24-2* | 137 | miR-425* | 197 | miR-7-1* |
| 18 | miR-1260b | 78 | miR-26a-1* | 138 | miR-4284 | 198 | miR-744* |
| 19 | miR-1262 | 79 | miR-26b* | 139 | miR-4286 | 199 | miR-766 |
| 20 | miR-1266 | 80 | miR-27a* | 140 | miR-4326 | 200 | miR-769-3p |
| 21 | miR-1271 | 81 | miR-27b* | 141 | miR-449b | 201 | miR-769-5p |
| 22 | miR-1275 | 82 | miR-28-3p | 142 | miR-454 | 202 | miR-873 |
| 23 | miR-1280 | 83 | miR-296-5p | 143 | miR-454* | 203 | miR-889 |
| 24 | miR-1287 | 84 | miR-29a* | 144 | miR-489 | 204 | miR-891a |
| 25 | miR-1294 | 85 | miR-29b-2* | 145 | miR-493 | 205 | miR-92a-1* |
| 26 | miR-1299 | 86 | miR-29c* | 146 | miR-499-3p | 206 | miR-93* |
| 27 | miR-1301 | 87 | miR-301b | 147 | miR-500a | 207 | miR-934 |
| 28 | miR-1303 | 88 | miR-30a* | 148 | miR-500a* | 208 | miR-937 |
| 29 | miR-130b* | 89 | miR-30b* | 149 | miR-500b | 209 | miR-941 |
| 30 | miR-132* | 90 | miR-30c-1* | 150 | miR-501-5p | 210 | miR-944 |
| 31 | miR-135b | 91 | miR-30c-2* | 151 | miR-502-5p | 211 | miR-99a* |
| 32 | miR-136* | 92 | miR-30d* | 152 | miR-504 | 212 | miR-99b* |
| 33 | miR-141* | 93 | miR-30e* | 153 | miR-511 | 213 | miR-10b |
| 34 | miR-143* | 94 | miR-3116 | 154 | miR-512-3p | 214 | miR-1260 |
| 35 | miR-144* | 95 | miR-3127 | 155 | miR-515-5p | 215 | miR-140-5p |
| 36 | miR-1468 | 96 | miR-3130-3p | 156 | miR-517a | 216 | miR-147b |
| 37 | miR-146b-3p | 97 | miR-3130-5p | 157 | miR-517b | 217 | miR-181c |
| 38 | miR-148a* | 98 | miR-3133 | 158 | miR-519a | 218 | miR-187 |
| 39 | miR-148b* | 99 | miR-3144-3p | 159 | miR-519c-3p | 219 | miR-188-3p |
| 40 | miR-1537 | 100 | miR-3158 | 160 | miR-520a-3p | 220 | miR-188-5p |
| 41 | miR-155* | 101 | miR-3182 | 161 | miR-548c-5p | 221 | miR-18a |
| 42 | miR-15a* | 102 | miR-3194 | 162 | miR-548d-5p | 222 | miR-190 |
| 43 | miR-15b* | 103 | miR-3196 | 163 | miR-548e | 223 | miR-190b |
| 44 | miR-16-1* | 104 | miR-32 | 164 | miR-548h | 224 | miR-19a |
| 45 | miR-16-2* | 105 | miR-32* | 165 | miR-548i | 225 | miR-219-1-3p |
| 46 | miR-17* | 106 | miR-3200-3p | 166 | miR-548j | 226 | miR-219-5p |
| 47 | miR-181a* | 107 | miR-335* | 167 | miR-548k | 227 | miR-301a |
| 48 | miR-181a-2* | 108 | miR-339-3p | 168 | miR-548n | 228 | miR-330-5p |
| 49 | miR-181c* | 109 | miR-33a* | 169 | miR-548o | 229 | miR-338-3p |
| 50 | miR-182* | 110 | miR-33b* | 170 | miR-548p | 230 | miR-34c-5p |
| 51 | miR-183* | 111 | miR-340* | 171 | miR-548y | 231 | miR-381 |
| 52 | miR-185* | 112 | miR-342-5p | 172 | miR-548z | 232 | miR-383 |
| 53 | miR-18a* | 113 | miR-34a* | 173 | miR-550a* | 233 | miR-424 |
| 54 | miR-192* | 114 | miR-34b* | 174 | miR-551a | 234 | miR-449a |
| 55 | miR-193b | 115 | miR-3605-5p | 175 | miR-576-3p | 235 | miR-450a |
| 56 | miR-195* | 116 | miR-3613-5p | 176 | miR-576-5p | 236 | miR-455-5p |
| 57 | miR-196a* | 117 | miR-3614-5p | 177 | miR-577 | 237 | miR-501-3p |
| 58 | miR-199b-5p | 118 | miR-3615 | 178 | miR-579 | 238 | miR-505 |
| 59 | miR-200a* | 119 | miR-365* | 179 | miR-580 | 239 | miR-542-3p |
| 60 | miR-200b* | 120 | miR-3656 | 180 | miR-582-3p | 240 | miR-582-5p |
| | | | | | | 241 | miR-9 |

3. The artificial milk product of claim 1, wherein said milk product comprises a colostrum product and a mature breast milk product.

4. The artificial milk product of claim 1, wherein said milk product comprises one or more characteristics selected from the following group:
(a1) two or more kinds of said exogenously added microRNAs are from Table A;
(a2) the varieties and contents of said exogenously added microRNAs are approximate or identical to the microRNA expression profiles of human colostrum;
(b1) two or more kinds of said exogenously added microRNAs are from Table B;
(b2) the varieties and contents of said exogenously added microRNAs are approximate or identical to the microRNA expression profiles of human mature breast milk;
(c1) two or more kinds of said exogenously added microRNAs are from Table C; and
(d1) two or more kinds of said exogenously added microRNAs are from Table D.

5. An artificial food supplement, wherein said food supplement contains bromatologically acceptable carriers and one or more human milk-derived microRNAs selected from the group consisting of:
(a) microRNAs specific to human colostrum and listed in Table A;
(b) microRNAs specific to human mature breast milk and listed in Table B;
(c) common microRNAs existing both in human colostrum and mature breast milk and listed in Table C; and
(d) microRNAs not existing in cow milk but existing in human colostrum or human mature breast milk, and listed in Table D.

6. The artificial food supplement of claim 5, wherein said food supplement has one or more characteristics selected from the following group:
(a1) two or more kinds of said exogenously added microRNAs are from Table A;
(a2) the varieties and contents of said exogenously added microRNAs are approximate or identical to the microRNA expression profiles of human colostrum;
(b1) two or more kinds of said exogenously added microRNAs are from Table B;
(b2) the varieties and contents of said exogenously added microRNAs are approximate or identical to the microRNA expression profiles of human mature breast milk;
(c1) two or more kinds of said exogenously added microRNAs are from Table C; and
(d1) two or more kinds of said exogenously added microRNAs are from Table D.

7. A method for identifying a milk product, wherein said method comprises:
(1) providing a milk product sample;
(2) detecting varieties and contents of microRNAs in said milk product sample; and
(3) comparing the varieties of microRNAs and contents thereof detected in step (2) with expression profiles of the microRNAs in a colostrum sample and a mature breast milk sample which are used as controls;
wherein, said milk product is identified as a colostrum product if the detected varieties of the microRNAs and the contents thereof are approximate or identical to the expression profiles of the microRNAs of the colostrum sample; and said milk product is identified as a mature breast milk product if the detected varieties of the microRNAs and the contents thereof are approximate or identical to the expression profiles of the microRNAs of the mature breast milk sample.

8. The method of claim 7, wherein said milk product comprises cow milk or a milk product derived therefrom, goat milk or a milk product derived therefrom, and human milk.

9. A method of manufacturing a milk product, wherein said method comprises:
(1) providing a milk product material;
(2) detecting varieties of microRNAs in said milk product material and contents thereof thereby obtaining a first microRNA expression profile;
(3) comparing the varieties of microRNAs and the contents thereof detected in step (2) with expression profiles of the microRNAs in a colostrum sample or a mature breast milk sample which are used as controls; and
(4) based on comparison results, adding one or more kinds of microRNAs to said milk product material and/or removing one or more kinds of microRNAs from said milk product material to produce the milk product, wherein the expression profiles of the microRNAs in said milk product are more approximate or identical to the expression profiles of the microRNAs in the colostrum sample or the mature breast milk sample which are used as controls.

10. A method of manufacturing a milk product, wherein said method comprises:
(1) providing a milk product material;
(2) adding one or more kinds of microRNAs to said milk product material and/or removing one or more kinds of microRNAs from said milk product material to produce the milk product, wherein the expression profiles of microRNAs in said milk product are more approximate or identical to the expression profiles of the microRNAs in a colostrum or a mature breast milk.

11. A method of manufacturing a food supplement, wherein said method comprises:
(1) detecting varieties and contents of the microRNAs in the breast milk of a certain subject to obtain a first microRNA expression profile of the subject;
(2) comparing the detected varieties of microRNAs of the first expression profile and the contents thereof with the expression profiles of microRNAs in a normal colostrum sample or a normal mature breast milk sample used as control, so as to determine microRNAs which are lack or insufficient in the first expression profile and needed to be supplemented;
(3) preparing the food supplement, wherein said food supplement contains the microRNAs as determined in the foregoing step, which are lack or insufficient in the first expression profile and needed to be supplemented.

12. An isolated microRNA set, wherein said microRNA set is composed of two or more kinds of human milk-derived microRNAs selected from the group consisting of:
(a) microRNAs specific to human colostrum and listed in Table A;
(b) microRNAs specific to human mature breast milk and listed in Table B;
(c) common microRNAs existing in both human colostrum and mature breast milk and listed in Table C; and
(d) microRNAs not existing in cow milk but existing in human colostrum or human mature breast milkand listed in Table D.

13. An artificial milk product, food or food supplement, wherein comprises the isolated microRNA set of claim 12 as an effective ingredient.
